# EUROPÄISCHE PATENTSCHRIFT

(11) **EP 0 730 009 B1**
(45) Veröffentlichungstag und Bekanntmachung des Hinweises auf die Patenterteilung: **16.05.2001**
(21) Anmeldenummer: 96101475.0
(22) Anmeldetag: 02.02.1996
(51) Int. Cl.: C09B 62/445, C09B 62/505

(54) **Faserreaktive Anthrachinonfarbstoffe**
Fiber reactive anthraquinon dyes
Colorants anthraquinoniques réactifs sur la fibre

(30) Priorität: 14.02.1995 DE 19504800
(43) Veröffentlichungstag der Anmeldung: 04.09.1996
(73) Patentinhaber: DyStar Textilfarben GmbH & Co. Deutschland KG, 60318 Frankfurt am Main (DE)
(72) Erfinder: Russ, Hubert Werner, Dr., D-65439 Flörsheim (DE); Schumacher, Christian, Dr., D-65779 Kelkheim (DE)

(56) Entgegenhaltungen:
- WO-A-94/29282

## Beschreibung

Die vorliegende Erfindung liegt auf dem technischen Gebiet der anthrachinoiden faserreaktiven Farbstoffe.

Anthrachinonfarbstoffe sind in der Literatur zahlreich beschrieben. Sie zeichnen sich durch ihre Brillanz, gute Lichtechtheiten und die Stabilität des Chromophors unter sowohl sauren als auch alkalischen Bedingungen aus. Jedoch haben sie den Nachteil der hohen Rohmaterial-Kosten und vergleichsweise niedriger Molextinktion. Aus diesem Grunde ist es beim Färben und Bedrucken von Substraten wichtig, daß die Farbstoffe sowohl überlegene Echtheiten, verbesserte Prozeß-Charakteristiken als auch kompetitive Kosten aufweisen.

Fast alle wichtigen anthrachinoiden Reaktivfarbstoffe sind Abkömmlinge der Bromaminsäure (4-Brom-1-amino-anthrachinon-2-sulfonsäure). Diese wird meist mit einer faserreaktiven Aminkomponente umgesetzt, wobei die faserreaktive Gruppe mit der Aminogruppe über aliphatische oder häufiger über aromatische Brückenglieder verknüpft sein kann (A.H.M. Renfrew, Rev. Prog. Color. Relat. Top. 15 (1985) 15).

Einer der wichtigsten bekannten Reaktivfarbstoffe zur Herstellung von brillanten, echten blauen Reaktivfärbungen auf Textilien ist C.I. Reactive Blue 19 der Formel (A) und wird erstmals in DE-A-965 902 erwähnt. Der Syntheseverlauf ist zahlreich in der Literatur beschrieben.

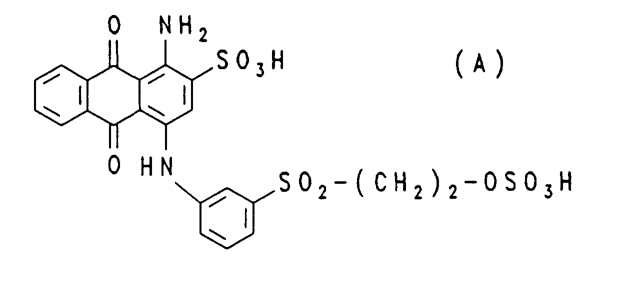

Die bekannten Anthrachinonfarbstoffe haben meist den Nachteil einer aufwendigen Synthese, wobei die Produkt- und Raum-Zeit-Ausbeute unbefriedigend sind. Andere brillante blaue Farbstoffe, beispielsweise Azofarbstoffe, bedürfen häufig einer Metallkomplexierung, um gute Echtheitseigenschaften zu erlangen, was ökologisch zunehmend bedenklich ist.

In der PCT/US 94/06727 werden Anthrachinonfarbstoffe beschrieben, die einen über eine p-Aminophenol-Einheit verknüpften faserreaktiven Triazinrest enthalten. Diese Farbstoffe besitzen jedoch Nachteile hinsichtlich Farbstärke und Herstellbarkeit.
Weitere faser reaktive Anthrachinonfarbstoffe sind in WO 94/29282 beschrieben.

Die Aufgabe der vorliegenden Erfindung war es, neue brillante, blaue Anthrachinonfarbstoffe mit hohen Echtheiten, insbesondere Chlorechtheit, Lichtechtheit und Waschechtheit, und guten Fixierausbeuten zur Verfügung zu stellen, die die genannten Nachteile des Standes der Technik überwinden.

Es wurde gefunden, daß die nachstehend definierten Verbindungen der Formel (1) überraschenderweise den gestellten Anforderungen gerecht werden.

Gegenstand der vorliegenden Erfindung sind Anthrachinonverbindungen der Formel (1), 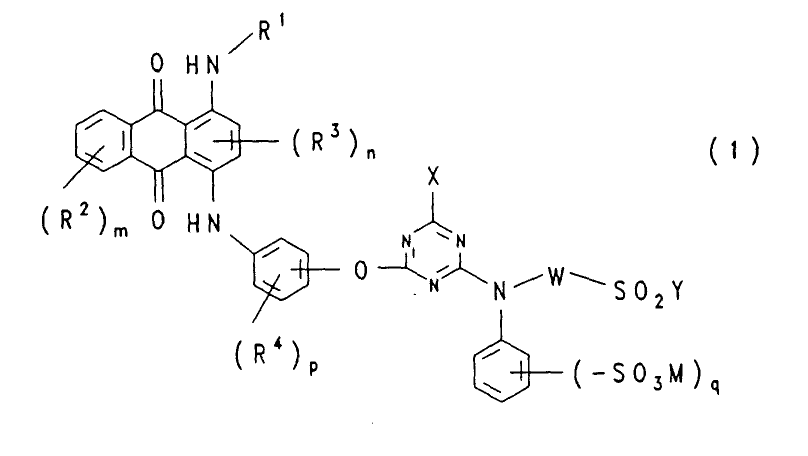 worin
- R¹: Wasserstoff, C₁-C₆-Alkyl-carbonyl, C₆-Arylcarbonyl, C₁-C₆-Alkyl, C₃-C₆-Cycloalkyl oder Phenyl, wobei C₁-C₆-Alkyl, C₃-C₆-Cycloalkyl und Phenyl durch einen oder mehrere Reste aus der Gruppe Hydroxy, Sulfo, Carboxy, C₁-C₄-Alkyl, C₁-C₄-Alkoxy, Halogen, Cyano, Amino und Nitro substituiert sein können;
- R²: Sulfo oder Carboxy;
- m: eine Zahl von 0 bis 2;
- R³: Sulfo, Carboxy oder Halogen, wie Chlor oder Brom;
- n: die Zahl 0 oder 1;
- R⁴: Sulfo, Carboxy, C₁-C₄-Alkyl oder C₁-C₄-Alkoxy;
- p: eine Zahl von 0 bis 2;
- W: eine C₁-C₆-Alkylengruppe;
- Y: Vinyl oder -CH₂CH₂-L, worin L eine unter alkalischen Bedingungen abspaltbare Gruppe ist;
- M: Wasserstoff oder Alkalimetall oder ein stöchiometrisches Equivalent eines Erdalkalimetalls;
- q: die Zahl 0 oder 1; und
- x: Halogen, Hydroxy, C₁-C₄-Alkyloxy, C₆-Aryloxy, Cyanamino, Amino, Carboxy-(C₁-C₄)alkyl-amino, C₁-C₄-Alkylamino, Di-(C₁-C₄-alkyl)-amino oder C₆-Arylamino, wobei die Alkyl- und Arylreste der besagten Alkylamino- und Arylamino-Gruppen unsubstituiert sind oder 1 bis 5 Substituenten aus der Gruppe Hydroxy, C₁-C₄-Alkoxy, Carboxy, Sulfo, Sulfato, C₁-C₄-Alkylsulfonyl, C₆-Arylsulfonyl, Halogen, Cyano und Nitro enthalten; oder der Rest eines heterocyclischen oder aliphatischen Amins, welches 1 bis 2 weitere Heteroatome aus der Gruppe N, O und S enthalten kann und mit einem Carboxyrest oder Aminocarbonylrest substituiert sein kann; oder die Gruppe -NR⁹R¹⁰R¹¹, worin R⁹, R¹⁰ und R¹¹ unabhängig voneinander Wasserstoff oder C₁-C₄-Alkyl bedeuten, ist.

Bevorzugt im Sinne der vorliegenden Erfindung sind Verbindungen der Formel (1), worin
- R¹: Wasserstoff, C₁-C₄-Alkyl, C₅-C₆-Cycloalkyl, Acetyl, Benzoyl, Phenyl oder Methylphenyl-carbonyl bedeutet, oder C₁-C₄-Alkyl, C₅-C₆-Cycloalkyl oder Phenyl, welche durch 1 bis 3 Reste aus der Gruppe Hydroxy, Sulfo, Carboxy, Methyl, Ethyl, Methoxy, Ethoxy, Fluor, Chlor, Brom, Cyano oder Nitro substituiert sind, bedeutet.

Insbesondere bevorzugt sind solche Verbindungen der Formel (1), worin
- R¹: Wasserstoff ist.

Bevorzugt sind weiterhin Verbindungen der Formel (1), worin
- R³: eine zur NH-R¹-Gruppe ortho-ständige Sulfogruppe ist.

Bevorzugt sind weiterhin Verbindungen der Formel (1), worin
- R⁴: Sulfo, Carboxy, Methyl, Ethyl, Methoxy oder Ethoxy ist.

Bevorzugt sind weiterhin Verbindungen der Formel (1), worin
- W: C₂-C₃-Alkylen, vorzugsweise 1,2-Ethylen oder 1,3-Propylen, ist.

Bevorzugt sind weiterhin Verbindungen der Formel (1), worin
- L: Chlor, Brom, -OSO₃M, -SSO₃M, -OPO₃M₂, vorzugsweise -OSO₃M oder Chlor, ist, wobei M Wasserstoff oder Alkalimetall bedeutet.

Bevorzugt sind weiterhin Verbindungen der Formel (1), worin
- m: die Zahl 0;
- n: die Zahl 1;
- p: die Zahl 0 oder 1; und
- q: die Zahl 0 sind.

Bevorzugt sind weiterhin Verbindungen der Formel (1), worin
- X: Fluor, Chlor, Cyanamino, Amino, Carboxymethylamino, β-Carboxyethylamino, β-Sulfoethylamino, N-Methyl-β-sulfo-ethylamino, β-Sulfatoethylamino, β-Hydroxyethylamino, Bis-(β-hydroxyethyl)-amino, Pyrrolidino, Piperidino, Piperazino, Morpholino, Pyridin-1-yl, 3-Carboxypyridin-1-yl, 3-Aminocarbonyl-pyridin-1-yl oder Trimethylammonium bedeutet.

Bevorzugt sind weiterhin Verbindungen der Formel (1), worin der substituierte -O-Triazinyl-Rest in para-Stellung zur NH-Gruppe des Phenylringes steht.

Besonders bevorzugte Farbstoffe sind Verbindungen der Formel (1a), (1b), (1c), (1d) und (1e) 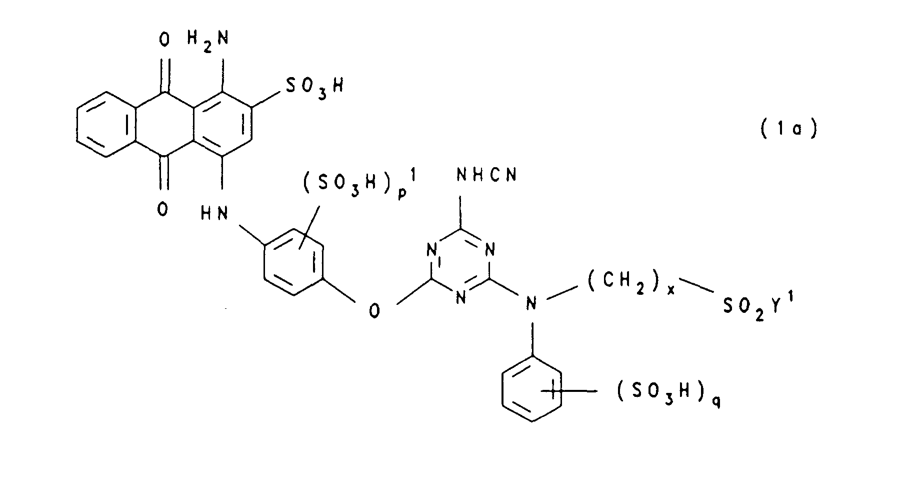 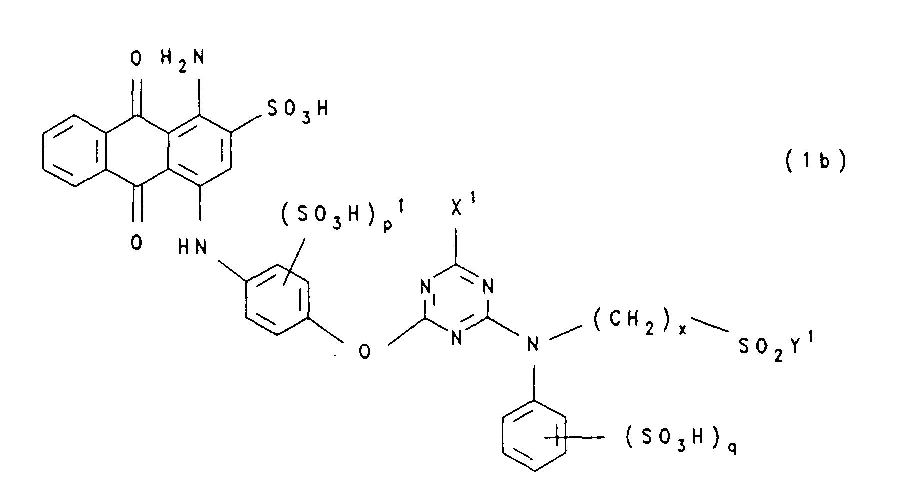 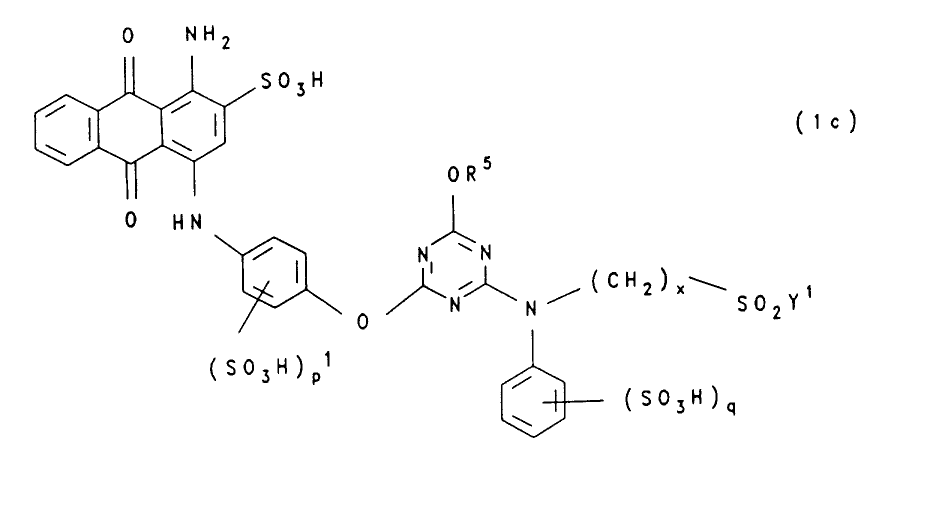 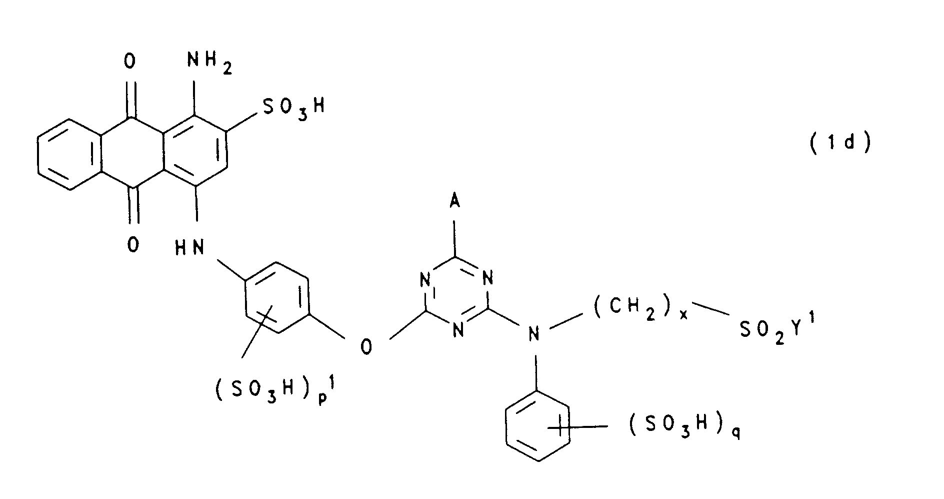 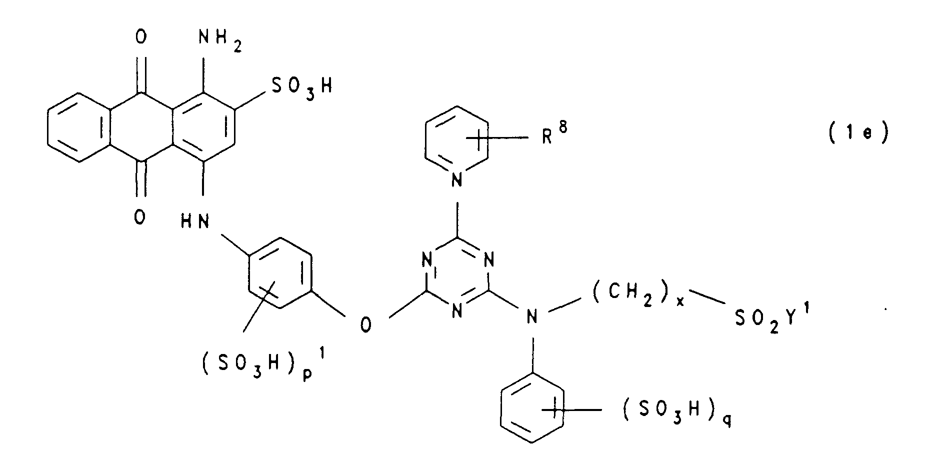 worin
- x: für 2 oder 3 steht;
- X¹: Chlor oder Fluor ist;
- R⁵: Methyl, Ethyl oder Phenyl ist;
- A: Amino, Carboxymethylamino, β-Carboxyethylamino, β-Sulfoethylamino, N-Methyl-β-Sulfoethylamino, β-Sulfatoethylamino, β-Hydroxyethylamino, Bis-(β-hydroxyethyl)-amino, Morpholino, Piperidino, Pyrrolidino oder Trimethylammonium ist,
- R⁸: Wasserstoff, Aminocarbonyl oder Carboxy ist; und
- Y¹: β-Sulfatoethyl, β-Chlorethyl oder Vinyl ist.

Insbesondere bevorzugt sind Farbstoffe der Formel (1f) 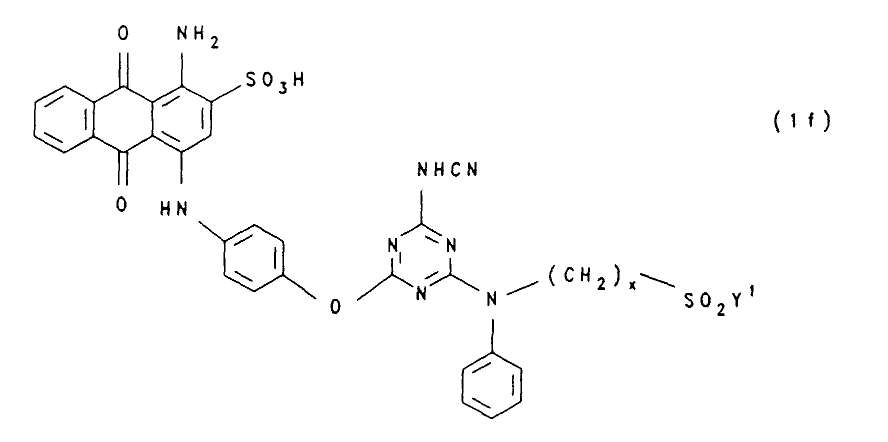 worin x und Y¹ eine der vorstehend genannten Bedeutungen haben.
Die bevorzugten Farbstoffe der Formeln (1a), (1c), (1d), (1e) und (1f) haben den Vorteil, daß sie kein organisch gebundenes Halogen besitzen, sofern Y¹ für β-Sulfatoethyl oder Vinyl steht, d.h. sie sind ökologisch besonders vorteilhaft.

Die vorliegende Erfindung betrifft weiterhin ein Verfahren zur Herstellung der erfindungsgemäßen Anthrachinonfarbstoffe (1), das dadurch gekennzeichnet ist, daß man eine Verbindung der Formel (2) 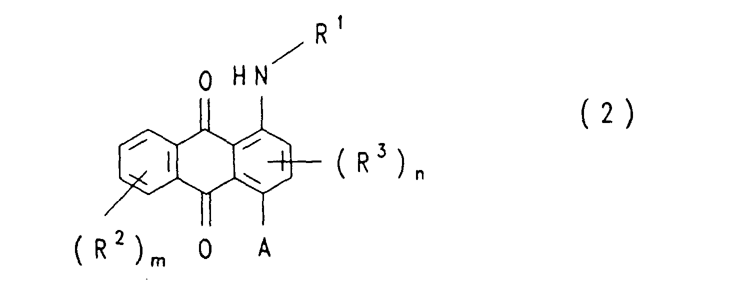 worin A für Fluor, Chlor, Brom, lod, Sulfo oder Nitro, insbesondere für Brom steht, und die Reste R¹, R² und R³ eine der vorstehend genannten Bedeutungen haben,
mit einer Verbindung der Formel (3), 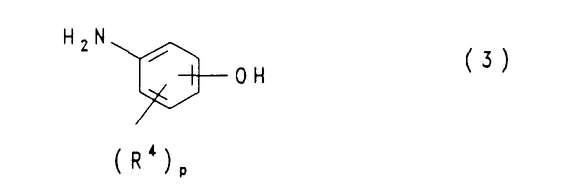 worin R⁴ und p eine der obengenannten Bedeutungen haben, in Gegenwart einer Cu(I)- oder Cu(II)-Verbindung oder einer Mischung davon, zu einer Verbindung der Formel (4) umsetzt 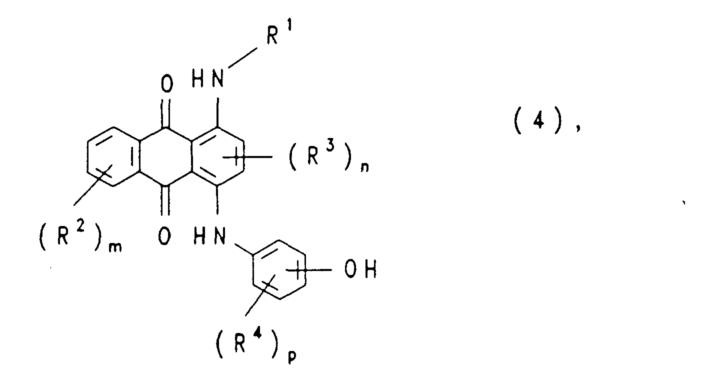 die Verbindung der Formel (4) entweder mit einer Verbindung der Formel (5) 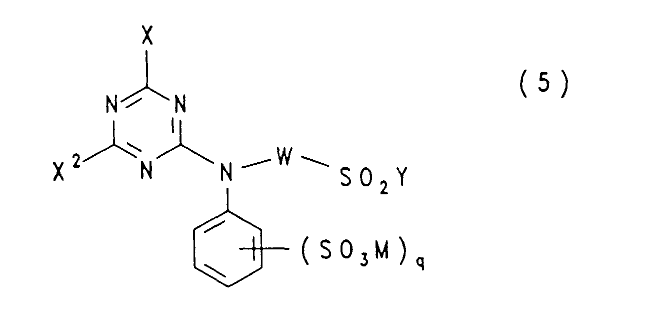 worin X, W, Y, M und q eine der vorstehend genannten Bedeutungen haben und X² für Halogen steht, zur Verbindung der Formel (1) umsetzt; oder die Verbindung der Formel (4) zunächst mit einer Verbindung der Formel (6) 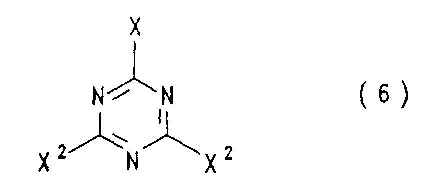 worin X und X² die vorstehend genannten Bedeutungen haben, zu einer Verbindung der Formel (7) umsetzt 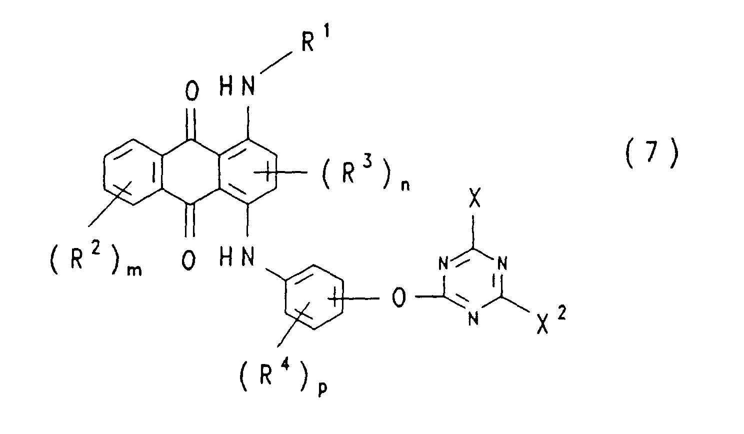 und die Verbindung der Formel (7) mit einem Amin der Formel (8) 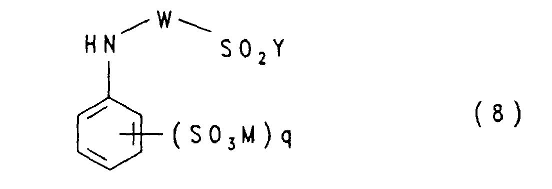 worin W, Y, M und q eine der vorstehend genannten Bedeutungen haben, zur Verbindung der Formel (1) umsetzt.

Die bevorzugte Verfahrensvariante zur Herstellung der Anthrachinonverbindungen der Formel (1) ist der vorstehend beschriebene Weg über die Verbindungen der Formeln (6), (7) und (8).

Es ist aus der Literatur bekannt, daß Ullmann-Kondensationen von Bromaminsäure mit aromatischen Aminen am besten in Gegenwart von Cu(I)-Verbindungen durchgeführt werden (J.Chem.Soc, Perkin II., 1974, 676 und Coll. Czech. Chem. Commun. 46 (1981), 92). Bevorzugt wird eine katalytische Menge, beispielsweise 0,5 bis 5 Gew.-%, bevorzugt 0,8 bis 2 Gew.-%, bezogen auf die Verbindung der Formel (2), einer Kupfer(I)-Verbindung, bevorzugt eines Cu(I)-Halogenids oder Cu(I)-Pseudohalogenids, wie CuCI, CuBr, Cul oder CuCN, verwendet und die Kondensation bei Temperaturen von 30 bis 90°C, bevorzugt 50 bis 70°C, und pH-Werten von 5 bis 10, bevorzugt 6 bis 9, durchgeführt.

Es wurde nun gefunden, daß die Kondensation der Verbindungen der Formeln (2) und (3) überraschenderweise auch in Gegenwart von Cu(II)-Verbindungen, wie Cu(II)-sulfat, Cu(II)-nitrat, Cu(II)-chlorid, Cu(II)-bromid, Cu(II)-carbonat oder Cu(II)-hydroxid, durchgeführt werden kann.

Gegenstand der vorliegenden Erfindung ist daher auch ein Verfahren zur Herstellung einer Verbindung der Formel (4) durch Kondensation einer Verbindung der Formel (2) mit einer Verbindung der Formel (3) in Gegenwart einer Cu(II)-Verbindung. Auch im Falle der Verwendung einer Cu(II)-Verbindung werden vorzugsweise die bei Verwendung einer Cu(I)-Vebindung genannten Mengen, Temperaturen und pH-Werte gewählt.

Sowohl bei Verwendung von Cu(I)- als auch von Cu(II)-Verbindungen wird die Kondensation in wäßrigem oder wäßrig-organischem Medium in Suspension oder in Lösung durchgeführt. Führt man die Reaktion in wäßrig-organischem Medium durch, so ist das organische Medium beispielsweise Aceton, Dimethylformamid, Dimethylsulfoxid oder N-Methylpyrrolidon. Vorteilhaft wird die bei der Kondensation freiwerdende Halogenwasserstoffsäure fortlaufend durch Zugabe wäßriger Alkalihydroxide, -carbonate, -phosphate, -silicate oder-bicarbonate neutralisiert. Die molaren Mengenverhältnisse der Verbindungen der Formel (2) und (3) betragen zweckmäßig 1:1 bis 1:1,4.

Die Umsetzung der Verbindung der Formel (4) mit der Verbindung der Formel (5) wird für den Fall, daß X² Chlor oder Brom ist, zweckmäßigerweise bei Temperaturen von 50 bis 100°C und pH-Werten von 3 bis 7 durchgeführt und für den Fall, daß X² Fluor ist, zweckmäßigerweise bei Temperaturen von -5 bis 50°C, vorzugsweise von 0 bis 25°C, und pH-Werten von 4 bis 10 durchgeführt. Die molaren Mengenverhältnisse der Verbindungen der Formeln (4) und (5) betragen zweckmäßigerweise 1:1 bis 1:1,5.

Die Umsetzung der Formel (4) mit der Verbindung der Formel (6) wird für den Fall, daß X² Chlor oder Brom ist, zweckmäßigerweise bei Temperaturen von 0 bis 50°C, bevorzugt 20 bis 40°C, und pH-Werten von 4 bis 10, bevorzugt 7 bis 9, durchgeführt und für den Fall, daß X² Fluor ist, zweckmäßigerweise bei Temperaturen von -5 bis 25°C, bevorzugt -2 bis + 5°C, und pH-Werten von 3 bis 5 durchgeführt. Die molaren Mengenverhältnisse der Verbindungen der Formeln (4) und (6) betragen zweckmäßigerweise 1:1 bis 1: 1,5.

Die Umsetzung der Verbindung der Formel (7) mit dem Amin der Formel (8) kann unter überraschend milden Bedingungen, bei Temperaturen von 30 bis 90°C, bevorzugt 40 bis 70°C, und pH-Werten von 4 bis 7, bevorzugt 5 bis 6, durchgeführt werden. Die molaren Mengenverhältnisse der Verbindungen der Formeln (7) und (8) betragen zweckmäßigerweise 1:1 bis 1:1,5.

Die erfindungsgemäßen Anthrachinonfarbstoffe der Formel (1) werden z.B. durch Ausrühren auf Eis/Wasser abgeschieden. Durch Neutralisieren mit Alkalihydroxid, -phosphat, -silicat, -carbonat oder -bicarbonat erhält man die wäßrigen Lösungen der Alkalisalze der Verbindungen der Formel (1). Die Isolierung der Farbstoffe aus dieser Lösung kann z.B. durch Aussalzen oder durch Sprühtrocknung erfolgen. Besonders vorteilhaft ist die direkte Verwendung der standardisierten wäßrigen Formulierungen, denen gegebenenfalls Puffersubstanzen zugesetzt werden und die gegebenenfalls aufkonzentriert werden können, für die entsprechenden anwendungstechnischen Zwecke.

Anthrachinonfarbstoffe der Formel (1), worin X für den Rest einer der vorstehend definierten Amine steht (X = X¹⁰), können auch in der Weise hergestellt werden, daß man zunächst den entsprechenden Anthrachinonfarbstoff der Formel (1), worin X für Chlor oder Fluor steht (X = X²⁰), nach einer der genannten Methoden herstellt und diesen dann mit dem entsprechenden Amin, bei Temperaturen von 40 bis 100°C und pH-Werten von 3 bis 8 in molaren Mengenverhältnissen von zweckmäßigerweise 1:1 bis 1:2 umsetzt.

Beispiele für solche genannten Amine sind:
Ammoniak, Methylamin, Ethylamin, Ethanolamin, Diethanolamin, β-Sulfoethylamin, N-Methyl-β-sulfoethylamin, β-Carboxyethylamin, Carboxymethylamin, 3-Carboxypyridin, 3-Aminocarbonylpyridin, Pyridin, Morpholin, Pyrrolidin, Piperidin, Piperazin, N'-Hydroxyethylpiperazin, N'-Sulfatoethylpiperazin, Anilin, 3-Sulfoanilin, 4-Sulfoanilin, 4-Carboxyanilin, 4-Methylanilin, 4-Methoxyanilin, 4-Methoxy-3-sulfo-anilin und 4-Methyl-3-sulfoanilin.

Gegenstand der vorliegenden Erfindung ist daher auch ein Verfahren zur Herstellung einer Anthrachinonverbindung der Formel (1A) 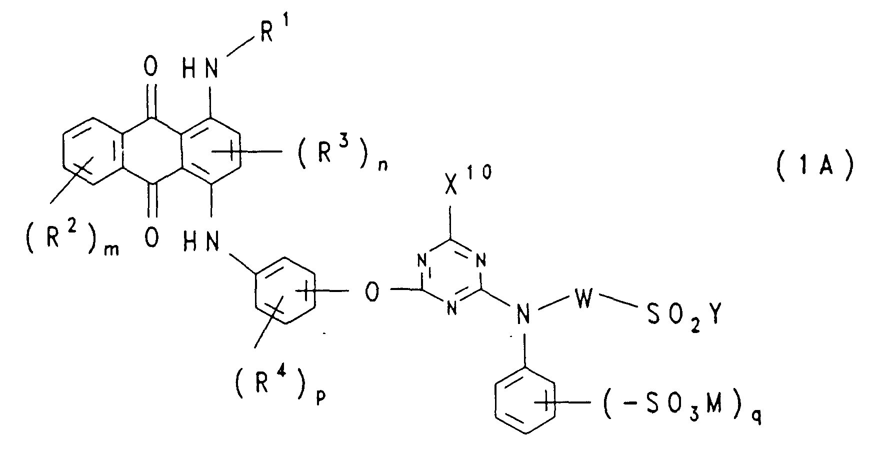 worin R¹, R², R³, R⁴, W, Y, M, m, n, p und q eine der in Anspruch 1 definierten Bedeutungen haben und X¹⁰ Amino, Cyanamino, Carboxy-(C₁-C₄)-alkyl-amino, C₁-C₄-Alkylamino, Di-(C₁-C₄-alkyl)amino, C₆-Arylamino, wobei die Alkyl- und Arylreste der besagten Alkylamino- und Arylamino-Gruppen unsubstituiert sind oder 1 bis 5 Substituenten aus der Gruppe Hydroxy, C₁-C₄-Alkoxy, Carboxy, Sulfo, Sulfato, C₁-C₄-Alkylsulfonyl, C₆-Arylsulfonyl, Halogen, Cyano und Nitro enthalten; oder der Rest eines heterocyclischen oder aliphatischen Amins, welches 1 bis 2 weitere Heteroatome aus der Gruppe N, O und S enthalten kann und mit einem Carboxyrest oder Aminocarbonylrest substituiert sein kann; oder die Gruppe -NR⁹R¹⁰R¹¹, worin R⁹, R¹⁰ und R¹¹ unabhängig voneinander Wasserstoff oder C₁-C₄-Alkyl bedeuten, ist,
dadurch gekennzeichnet, daß man eine Anthrachinonverbindung der Formel (1B) 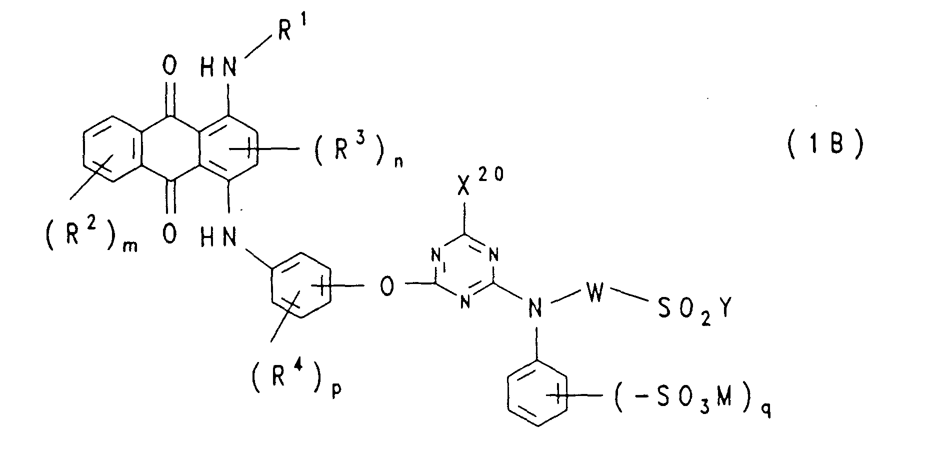 worin X²⁰ Chlor oder Fluor bedeutet, mit Ammoniak, H₂NCN, einem Carboxy-(C₁-C₄)-alkylamin, C₁-C₄-Alkylamin, Di-(C₁-C₄-alkyl)-amin, C₆-Arylamin, wobei die Alkyl- und Arylreste der besagten Alkylamino- und Arylamino-Gruppen unsubstituiert sind oder 1 bis 5 Substituenten aus der Gruppe Hydroxy, C₁-C₄-Alkoxy, Carboxy, Sulfo, Sulfato, C₁-C₄-Alkylsulfonyl, C₆-Arylsulfonyl, Halogen, Cyano und Nitro enthalten;
oder mit einem heterocyclischen oder aliphatischen Amin, welches 1 bis 2 weitere Heteroatome aus der Gruppe N, O und S enthalten kann und mit einem Carboxyrest oder Aminocarbonylrest substituiert sein kann;
oder mit einem Mono-, Di- oder Tri-(C₁-C₄-alkyl)-ammonium-Salz umsetzt.

Verbindungen der Formel (2) sind beispielsweise:
1-Amino-4-brom-, 1-Amino-4-brom-2-sulfo-, 1-Amino-2,4-dibrom-,
1-Methylamin-2-carboxy-4-brom-, 1-Ethylamino-2-carboxy-4-brom-,
1-Isopropylamino-2-carboxy-4-brom-, 1-Amino-4-brom-2,6-disulfo-,
1-Amino-4-brom-2,7-disulfo-, 1-Amino-4-brom-2,5,8-trisulfo-,
1-Propylamino-4-brom-2-sulfo-, 1-Amino-4-brom-2-carboxy-,
1-Amino-4-brom-2-sulfo-6-carboxy-, 1-Amino-4-brom-2-sulfo-7-carboxy-,
1-Methylamino-4-brom-, 1-Methylamino-4-brom-2-sulfo-, 1-Ethylamino-4-brom-2-sulfo-, 1-(β-Hydroxyethyl)amino-4-bromo-2-sulfo-, 1-Phenylamino-4-brom-2-sulfo-, 1-Amino-2-brom-4-nitro-, 1-Cyclohexylamino-4-brom-,
1-Benzoylamino-4-brom-, 1-Acetylamino-4-brom-, 1-Cyclohexylamino-4-brom-5-sulfo-, 1-Cyclohexylamino-4-brom-6-sulfo-, 1-Cyclohexylamino-4-brom-7-sulfo- oder 1-Cyclohexylamino-4-brom-8-sulfo-Anthrachinon.
Besonders bevorzugt ist als Anthrachinon-Komponente 1-Amino-4-brom-2-sulfoanthrachinon. Die Herstellung der genannten Anthrachinonverbindungen ist dem Fachmann bekannt und beispielsweise in Houben-Weyl, Bd. 7/3c, 4. Auflage, S. 46 ff, beschrieben.

Verbindungen der Formel (3) sind beispielsweise 4-Aminophenol, 4-Aminophenol-2-sulfonsäure, 4-Aminophenol-3-sulfosäure, 3-Aminophenol, 3-Aminophenol-4-sulfosäure, 3-Aminophenol-5-sulfosäure, 2-Carboxy-4-aminophenol und 3-Carboxy-4-aminophenol. Die Herstellung solcher Aminophenole ist dem Fachmann bekannt und beispielsweise in Houben-Weyl, Bd. XI/1 beschrieben.

Verbindungen der Formel (5) können beispielsweise durch Umsetzung von Verbindungen der Formel (6) mit Verbindungen der Formel (8) hergestellt werden (EP-A1-0 629 667).

Verbindungen der Formel (6) sind beispielsweise 2,4,6-Trichlor-1,3,5-triazin, 2,4,6-Trifluor-1,3,5-triazin, 2-Cyanamino-4,6-dichlor-1,3,5-triazin, 2-Methoxy-4,6-dichlor-1,3,5-triazin, 2-Ethoxy-4,6-dichlor-1,3,5-triazin, 2-Phenoxy-4,6-dichlor-1,3,5-triazin und 2-Amino-4,6-dichlor-1,3,5-triazin. Die Herstellung solcher Triazine ist dem Fachmann bekannt und beispielsweise in DE-A-2 756 438 und DE-A-3 930 704 beschrieben.

Verbindungen der Formel (8) sind beispielsweise 3-(N-Phenyl)-aminopropyl-2'-sulfatoethyl-sulfon, 3-N-[3"-Sulfophenyl]aminopropyl-2'-sulfatoethyl-sulfon, 3-N-[4"-Sulfophenyl]aminopropyl-2'-sulfatoethyl-sulfon, 3-N-[2"-Sulfophenyl]-aminopropyl-2'-sulfatoethyl-sulfon, 2-(N-Phenyl)-aminoethyl-2'-sulfatoethylsulfon, 2-N-[2"-Sulfophenyl]aminoethyl-2'-sulfatoethyl-sulfon, 2-N-[3"-Sulfophenyl]aminoethyl-2'-sulfatoethyl-sulfon und 2-N-[4"-Sulfophenyl]-aminoethyl-2'-sulfatoethyl-sulfon.
Die Herstellung solcher Verbindungen ist beispielsweise in EP-A1-0 629 667 und EP-A1-0 568 876 beschrieben.

Die Verbindungen der Formel (1) haben faserreaktive Eigenschaften und besitzen sehr gute Farbstoffeigenschaften. Sie können zum Färben und Bedrucken von hydroxy-, mercapto-, amino- und/oder carbonamidgruppenhaltigen Materialien, insbesondere Fasermaterialien, verwendet werden. Es werden brillante blaue Farbtöne mit hervorragenden Echtheiten, wie Lichtechtheit, Waschechtheit und Chlorwasserechtheit, erhalten, ohne daß Metallkomplexierungen erforderlich sind. Die erfindungsgemäßen Farbstoffe sind somit ökologisch von Vorteil.

Gegenstand der vorliegenden Erfindung ist deshalb auch die Verwendung der Verbindungen der Formel (1) zum Färben oder Bedrucken der genannten Materialien. Dabei wird die Verbindung der Formel (1) auf das Material aufgebracht oder in das Material eingebracht und auf oder in dem Material mittels Wärme oder mit Hilfe eines alkalisch wirkenden Mittels fixiert.

Beispiele für geeignete Materialien sind native oder regenerierte Cellulosematerialien, wie Baumwolle, Leinen, Viskose, Rayon, chemisch modifizierte Cellulosefasern, beispielsweise durch Aminoverbindungen modifizierte Celluloserfasern, proteinische Fasern, wie Wolle oder Seide, und synthetische Polyamide, wie Nylon oder Perlon, die alle dem Fachmann hinreichend bekannt sind.

Geeignete Applikationsverfahren sind z.B. das Ausziehverfahren in Haspelkufen, Jets oder kontinuierliche Färbetechniken. Insbesondere geeignet sind die erfindungsgemäßen Farbstoffe zum Färben und Bedrucken von Cellulosematerialien durch Verwendung einer kurzflottigen Applikationstechnik, z.B. bei einem Flottenverhältnis von 1:0,4 bis 1:5, sowie für den Textildruck oder Klotz-Färbeverfahren, wie beispielsweise Kontinue-Verfahren.

Insbesondere geeignet sind die bevorzugten Farbstoffe (1a), (1c), (1d), (1e) und besonders (1f) für Textildruckverfahren. Die bevorzugten Farbstoffe (1a), (1c), (1d), (1e) und besonders (1f) sind weiterhin dadurch gekennzeichnet, daß sie im Ätzdruck-Verfahren vorteilhaft appliziert werden können.

Die Farbstoffe der vorliegenden Erfindung sind in dieser Beschreibung in Form ihrer freien Säuren geschrieben. Sie können aber auch als Salze dieser Säuren angewendet werden. Bevorzugt werden sie in ihrer Salzform und besonders bevorzugt in der Alkalimetall- und Erdalkalimetallsalz-Form verwendet wie z.B. als Na-, K- oder Li-Salze.

In den Beispielen bedeuten Prozentangaben Gewichtsprozente und Teile Gewichtsteile. Die angegebenen Absorptionsmaxima (Lambda max) im sichtbaren Bereich wurden anhand ihrer Alkalimetallsalze in wäßriger Lösung ermittelt.

### Beispiel A

38,2 Teile 1-Amino-4-brom-2-sulfo-anthrachinon (Bromaminsäure) werden in wäßriger Suspension mit 12,7 Teilen 4-Aminophenol unter Zusatz von 1 Teil Kupfer(II)-sulfat bei einem pH-Wert von 8,5 und einer Temperatur von 70°C innerhalb 1 bis 2 Stunden kondensiert. Hierbei entsteht eine dunkelblaue Lösung. Nach Abkühlen auf 20-25°C wird angesäuert, bis ein pH-Wert von ca. 1 erreicht ist, ca. 1h bei 60°C gerührt, mit Natriumchlorid ausgesalzen und der Farbstoff-Chromophor 4(4'-Hydroxyphenyl)amino-1-amino-2-sulfo-anthrachinon durch Absaugen isoliert.

Die so erhaltene Anthrachinon-Verbindung hat die Formel

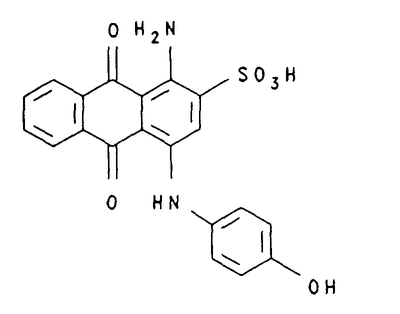

### Beispiel 1

41 Teile der Anthrachinon-Verbindung aus Beispiel A werden in 200 Teilen Wasser bei einem pH-Wert von 8,5 und einer Temperatur von 35°C gelöst, anschließend mit 20 Teilen 2-Cyanamino-4,6-dichlor-1,3,5-triazin versetzt und sodann bei einem pH-Wert von 8 bis 9 und einer Temperatur von 35°C kondensiert. Es wird einige Zeit gerührt, bis die Umsetzung beendet ist, und dann mit 36 Teilen 3-(N-Phenyl)-aminopropyl-2'-sulfatoethyl-sulfon versetzt. Bei einer Temperatur von 50°C und pH 8,5 wird die Umsetzung innert ca. 3h durchgeführt. Anschließend wird das Produkt mit Kaliumchlorid ausgesalzen, oder bevorzugt durch Eindampfen im Vakuum, isoliert. Man erhält 85 Teile des Farbstoffs der Formel 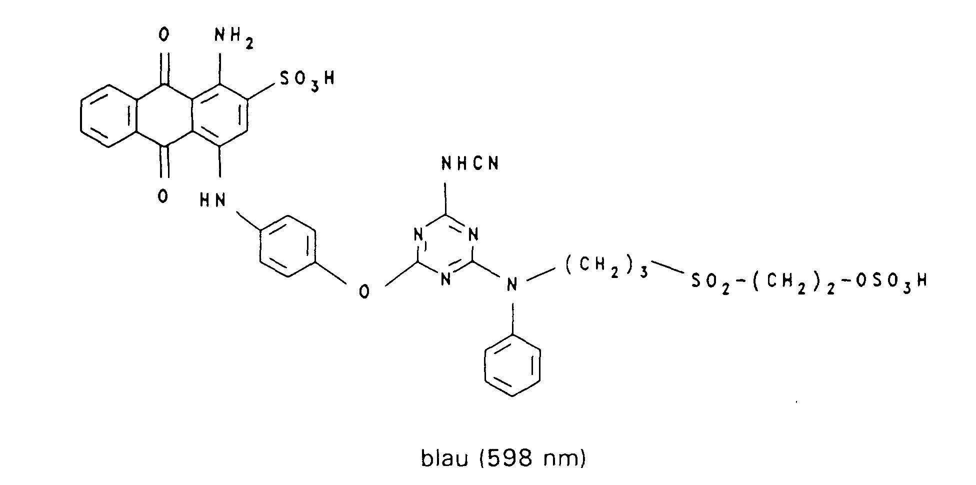 mit einer Reinheit von 90 % per HPLC.
Der Farbstoff färbt und bedruckt Cellulosefasern in brillanten blauen Farbtönen mit hohen Farbstärken bei guter Egalität und sehr guten Echtheitseigenschaften, insbesondere Lichtechtheiten.

### Beispiel 2

41 Teile der Anthrachinon-Verbindung aus Beispiel A werden in 200 Teilen Wasser bei einem pH-Wert von 8,5 und einer Temperatur von 40°C gelöst, anschließend mit 20 Teilen 2-Cyanamino-4,6-dichlor-1,3,5-triazin versetzt und sodann bei einem pH-Wert von 8 bis 9 und einer Temperatur von 40°C kondensiert. Es wird einige Zeit gerührt, bis die Umsetzung beendet ist und dann mit 33 Teilen 2-(N-Phenyl)-aminoethyl-2'-sulfatoethyl-sulfon versetzt. Bei einer Temperatur von 50°C und pH 8,5 wird die Umsetzung innert ca. 3h durchgeführt. Anschließend wird das Produkt mit Kaliumchlorid ausgesalzen, oder bevorzugt durch Eindampfen im Vakuum isoliert. 82 Teile des Farbstoffs der Formel 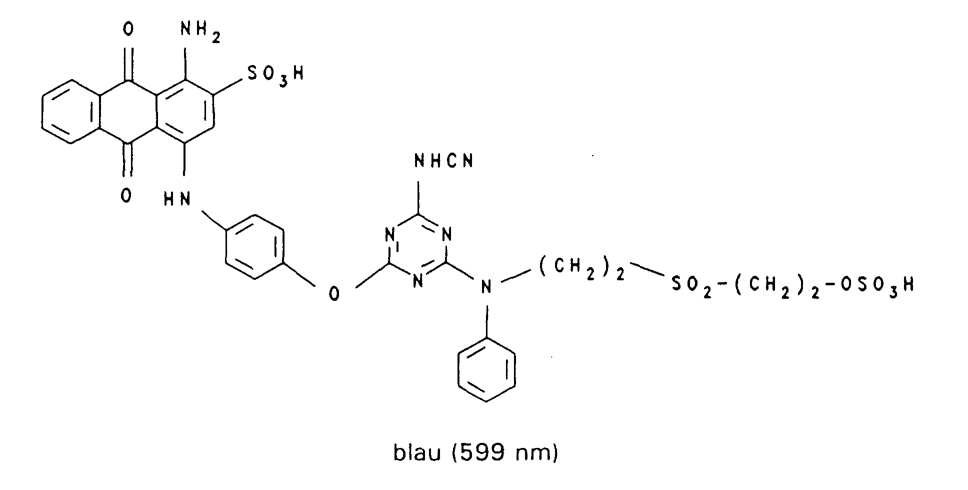 werden mit einer Reinheit von 84 % per HPLC erhalten.

Der Farbstoff färbt und bedruckt Cellulosefasern in brillanten blauen Farbtönen mit hohen Farbstärken bei guter Egalität und sehr guten Echtheitseigenschaften, insbesondere Lichtechtheiten.

### Beispiel 3

41 Teile der Anthrachinon-Verbindung aus Beispiel A werden in 200 Teilen Wasser bei einem pH-Wert von 8,5 und einer Temperatur von 40°C gelöst, anschließend mit 20 Teilen 2,4,6-Trichlor-1,3,5-triazin versetzt und sodann bei einem pH-Wert von 7 bis 8 und einer Temperatur von 0 bis 5°C kondensiert. Es wird einige Zeit gerührt, bis die Umsetzung beendet ist und dann mit 33 Teilen N-Phenyl-2-[(2'-sulfatoethyl)sulfonyl]-ethylamin versetzt.
Bei einer Temperatur von 50°C und pH 8,5 wird die Umsetzung innert ca. 3h durchgeführt. Anschließend wird das Produkt mit Kaliumchlorid ausgesalzen, oder bevorzugt durch Eindampfen im Vakuum isoliert. 82 Teile des Farbstoffs der Formel 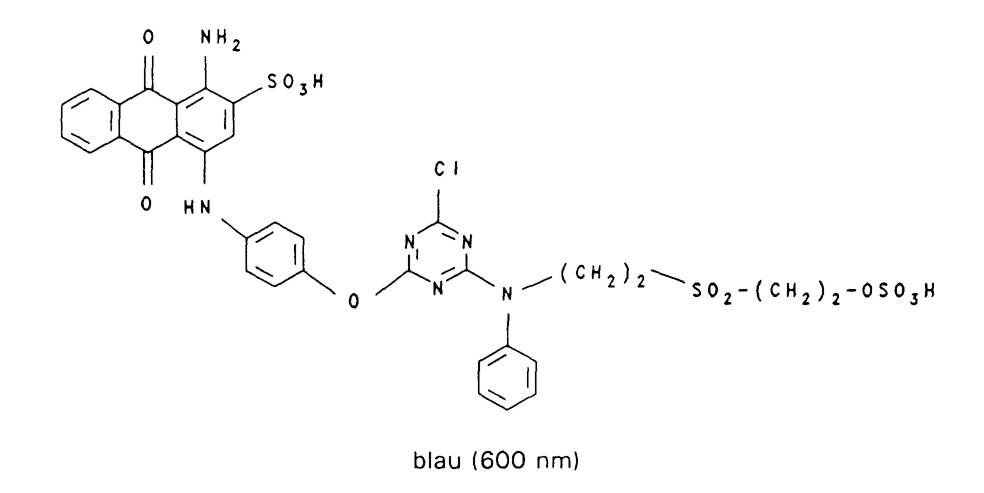 werden in einer Reinheit von 87 % per HPLC erhalten.
Der Farbstoff färbt und bedruckt Cellulosefasern in brillanten blauen Farbtönen mit hohen Farbstärken bei guter Egalität und sehr guten Echtheitseigenschaften, insbesondere Lichtechtheiten.

### Beispiel 4

87 Teile der Verbindung aus Beispiel 3 werden in 300 Teilen Wasser bei einem pH-Wert von 6,5 und einer Temperatur von 30°C gelöst, anschließend mit 8,5 Teilen Morpholin versetzt und sodann bei einem pH-Wert von 7 bis 8 und einer Temperatur von 60°C kondensiert. Es wird einige Zeit gerührt, bis die Umsetzung beendet ist. Anschließend wird das Produkt mit Kaliumchlorid ausgesalzen, oder bevorzugt durch Eindampfen im Vakuum isoliert. 85 Teile des Farbstoffs der Formel 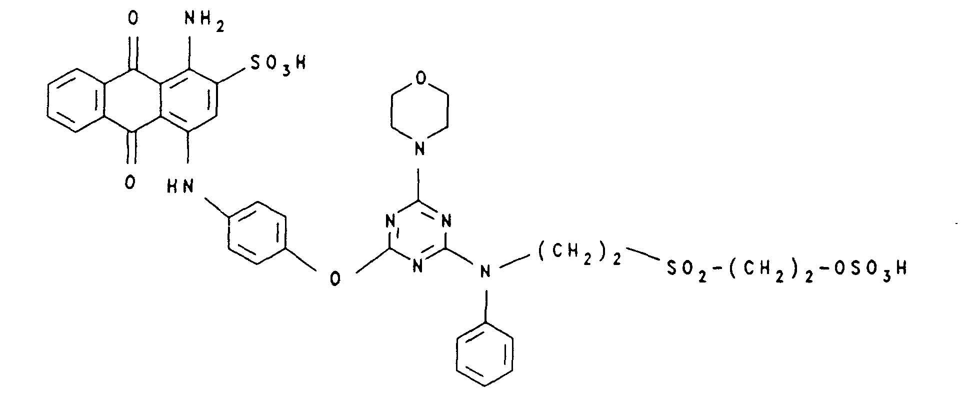 werden in einer Reinheit von 73 % per HPLC erhalten.
Der Farbstoff färbt und bedruckt Cellulosefasern in brillanten blauen Farbtönen mit hohen Farbstärken bei guter Egalität und sehr guten Echtheitseigenschaften, insbesondere Lichtechtheiten.

### Beispiel 5

41 Teile der Anthrachinon-Verbindung aus Beispiel A werden in 500 Teilen Wasser bei einem pH-Wert von 8 und einer Temperatur von 30°C gelöst, sodann auf 0 bis 5°C gekühlt, anschließend mit 45 Teilen der aus der EP-A-0 568 876 bekannten Verbindung 2-[N-Phenyl-(2'-sulfatoethyl-sulfonyl)-ethyl-aminol-4,6-difluor-1,3,5-triazin versetzt und sodann bei einem pH-Wert von 7 bis 8 und einer Temperatur von 10 bis 20°C kondensiert. Es wird einige Zeit gerührt, bis die Umsetzung beendet ist. Anschließend wird das Produkt mit Kaliumchlorid ausgesalzen, oder bevorzugt durch Eindampfen im Vakuum isoliert. 80 Teile des Farbstoffs der Formel 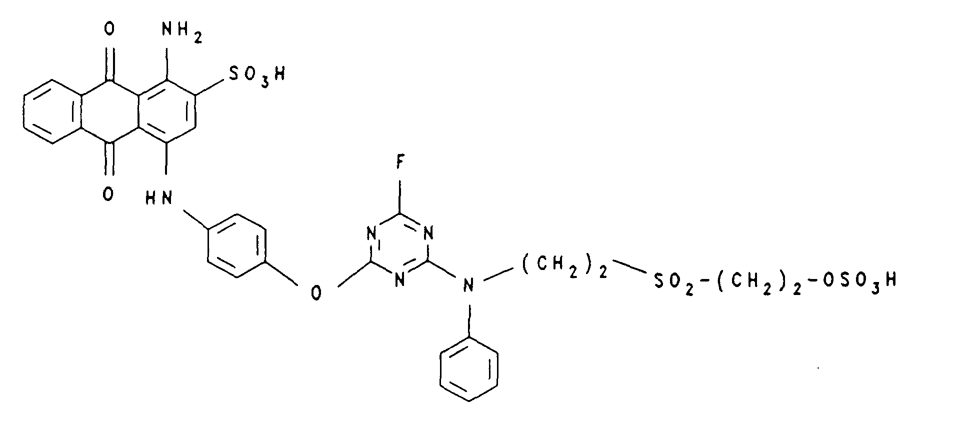 werden in einer Reinheit von 88 % per HPLC erhalten.
Der Farbstoff färbt und bedruckt Cellulosefasern in brillanten blauen Farbtönen mit hohen Farbstärken bei guter Egalität und sehr guten Echtheitseigenschaften, insbesondere Lichtechtheiten.

### Beispiel 6

80 Teile der Verbindung aus Beispiel 5 werden in 300 Teilen Wasser bei einem pH-Wert von 6,5 und einer Temperatur von 30°C gelöst, anschließend mit 12 Teilen Nicotinsäure versetzt und sodann bei einem pH-Wert von 3 bis 4 und einer Temperatur von 70 bis 80°C kondensiert. Es wird einige Zeit gerührt, bis die Umsetzung beendet ist. Anschließend wird das Produkt mit Kaliumchlorid ausgesalzen, oder bevorzugt durch Eindampfen im Vakuum isoliert. 90 Teile des Farbstoffs der Formel 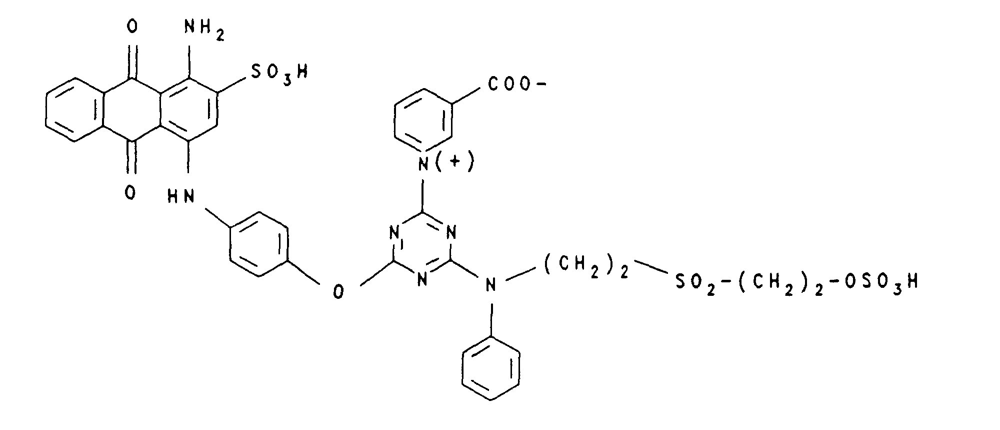 werden in einer Reinheit von 73 % per HPLC erhalten.
Der Farbstoff färbt und bedruckt Cellulosefasern in brillanten blauen Farbtönen mit hohen Farbstärken bei guter Egalität und sehr guten Echtheitseigenschaften, insbesondere Lichtechtheiten.

Weitere erfindungsgemäße Farbstoffe der folgenden Formel werden erhalten, wenn man analog zu Beispiel 1 verfährt und die aus der Tabelle ersichtlichen entsprechenden Vorprodukte für die Synthese einsetzt.

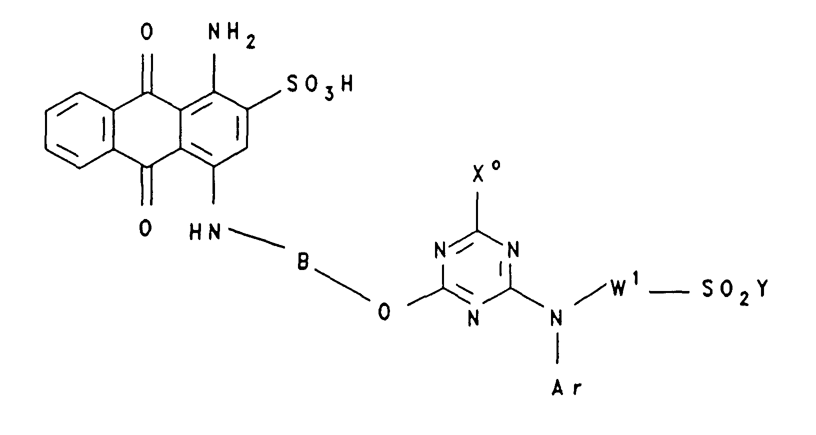

| Bsp. Nr. | B(1 =Aminoverknüpfung) | X° | N(Ar)-W¹-SO₂Y | Farbton auf Baumwolle |
|---|---|---|---|---|
| 7 | 1,4-Phenylen | Chlor | N-Phenyl-[3'-(β-sulfatoethyl)-sulfonylpropyl]-amino | blau (602) |
| 8 | dito | Cyanamino | N-(3"-Sulfophenyl)-[3'-(β-sulfatoethyl)-sulfonyl-propyl]-amino | blau (604) |
| 9 | dito | Cyanamino | N-(3"-Sulfophenyl)-[2'-(β-sulfatoethyl)-sulfonyl-ethyl]-amino | blau (605) |
| 10 | 1,3-Phenylen | Fluor | N-Phenyl-[3'-(β-sulfatoethyl)-sulfonyl-propyl]-amino | blau (605) |
| 11 | 3-Sulfo-1,4-phenylen | Amino | dito | blau (606) |
| 12 | 3-Sulfo-1,4-phenylen | Piperidino | dito | blau (603) |
| 13 | 4-Sulfo-1,3-phenylen | Pyrrolidino | dito | blau (603) |
| 14 | 1,4-Phenylen | (3-Aminocarbonyl)-pyridinyl | N-Phenyl-[3'-(β-sulfatoethyl)-sulfonylpropyl]-amino | blau (603) |
| 15 | dito | Methoxy | N-Phenyl-[3'-(β-sulfatoethyl)-sulfonylpropyl]-amino | blau (603) |
| 16 | dito | Phenoxy | N-Phenyl-[3'-(β-sulfatoethyl)-sulfonylpropyl]-amino | blau (603) |
| | | | | |

## Patentansprüche

1. Anthrachinonverbindung der Formel (1) 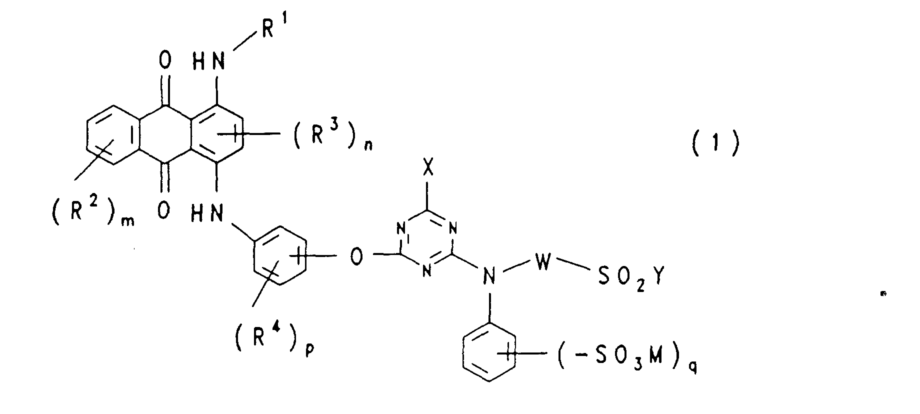 worin
R¹ Wasserstoff, C₁-C₆-Alkyl-carbonyl, C₆-Arylcarbonyl, C₁-C₆-Alkyl, C₃-C₆-Cycloalkyl oder Phenyl, wobei C₁-C₆-Alkyl, C₃-C₆-Cycloalkyl und Phenyl durch einen oder mehrere Reste aus der Gruppe Hydroxy, Sulfo, Carboxy, C₁-C₄-Alkyl, C₁-C₄-Alkoxy, Halogen, Cyano, Amino und Nitro substituiert sein können;
R² Sulfo oder Carboxy;
m eine Zahl von 0 bis 2;
R³ Sulfo, Carboxy oder Halogen;
n die Zahl 0 oder 1;
R⁴ Sulfo, Carboxy, C₁-C₄-Alkyl oder C₁-C₄-Alkoxy;
p eine Zahl von 0 bis 2;
W eine C₁-C₆-Alkylengruppe;
Y Vinyl oder -CH₂CH₂-L, worin L eine unter alkalischen Bedingungen abspaltbare Gruppe ist;
M Wasserstoff oder Alkalimetall oder ein stöchiometrisches Equivalent eines Erdalkalimetalls;
q die Zahl 0 oder 1; und
X Halogen, Hydroxy, C₁-C₄-Alkyloxy, C₆-Aryloxy, Cyanamino, Amino, Carboxy-(C₁-C₄)-alkyl-amino, C₁-C₄-Alkylamino, Di-(C₁-C₄-alkyl)-amino oder C₆-Arylamino, wobei die Alkyl- und Arylreste der besagten Alkylamino- und Arylamino-Gruppen unsubstituiert sind oder 1 bis 5 Substituenten aus der Gruppe Hydroxy, C₁-C₄-Alkoxy, Carboxy, Sulfo, Sulfato, C₁-C₄-Alkylsulfonyl, C₆-Arylsulfonyl, Halogen, Cyano und Nitro enthalten; oder der Rest eines heterocyclischen oder aliphatischen Amins, welches 1 bis 2 weitere Heteroatome aus der Gruppe N, O und S enthalten kann und mit einem Carboxyrest oder Aminocarbonylrest substituiert sein kann; oder die Gruppe -NR⁹R¹⁰R¹¹, worin R⁹, R¹⁰ und R¹¹ unabhängig voneinander Wasserstoff oder C₁-C₄-Alkyl bedeuten, ist.

2. Anthrachinonverbindung nach Anspruch 1, dadurch gekennzeichnet, daß
R¹ Wasserstoff, C₁-C₄-Alkyl, C₅-C₆-Cycloalkyl, Acetyl, Benzoyl, Phenyl oder Methylphenyl-carbonyl bedeutet, oder C₁-C₄-Alkyl, C₅-C₆-Cycloalkyl und Phenyl, welche durch 1 bis 3 Reste aus der Gruppe Hydroxy, Sulfo, Carboxy, Methyl, Ethyl, Methoxy, Ethoxy, Fluor, Chlor, Brom, Cyano oder Nitro substituiert sind, bedeutet.

3. Anthrachinonverbindung nach Anspruch 1 oder 2, dadurch gekennzeichnet, daß R³ Chlor, Brom oder eine zur NH-R¹-Gruppe ortho-ständige Sulfogruppe ist.

4. Anthrachinonverbindung nach mindestens einem der Ansprüche 1 bis 3, dadurch gekennzeichnet, daß W C₂-C₃-Alkylen, vorzugsweise 1,2-Ethylen oder 1,3-Propylen, ist.

5. Anthrachinonverbindung nach mindestens einem der Ansprüche 1 bis 4, dadurch gekennzeichnet, daß L Chlor, Brom, -OSO₃M, -SSO₃M oder -OPO₃M₂ ist, wobei M Wasserstoff oder Alkalimetall bedeutet.

6. Anthrachinonverbindung nach mindestens einem der Ansprüche 1 bis 5,
dadurch gekennzeichnet, daß
m die Zahl 0;
n die Zahl 1;
p die Zahl 0 oder 1 und
q die Zahl 0 ist.

7. Anthrachinonverbindung nach mindestens einem der Ansprüche 1 bis 6,
dadurch gekennzeichnet, daß
X Fluor, Chlor, Cyanoamino, Amino, Carboxymethylamino, β-Carboxyethylamino, β-Sulfoethylamino, N-Methyl-β-sulfoethylamino, β-Sulfatoethylamino, β-Hydroxyethylamino, Bis-(β-hydroxyethyl)-amino, Pyrrolidino, Piperidino, Piperazino, Morpholino, Pyridin-1-yl, 3-Carboxy-pyridin-1-yl, 3-Aminocarbonyl-pyridin-1-yl oder Trimethylammonium bedeutet.

8. Anthrachinonverbindung nach Anspruch 1, gekennzeichnet durch die Formeln (1a), (1b), (1c), (1d), (1e) oder (1f) 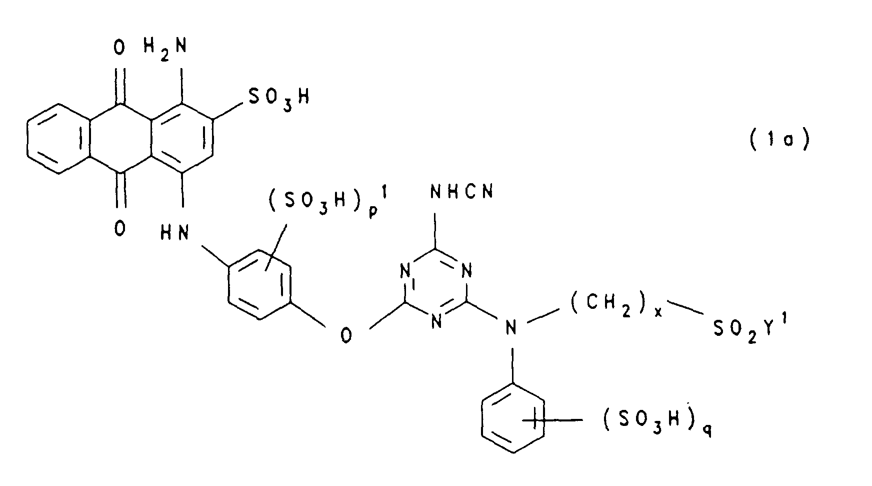 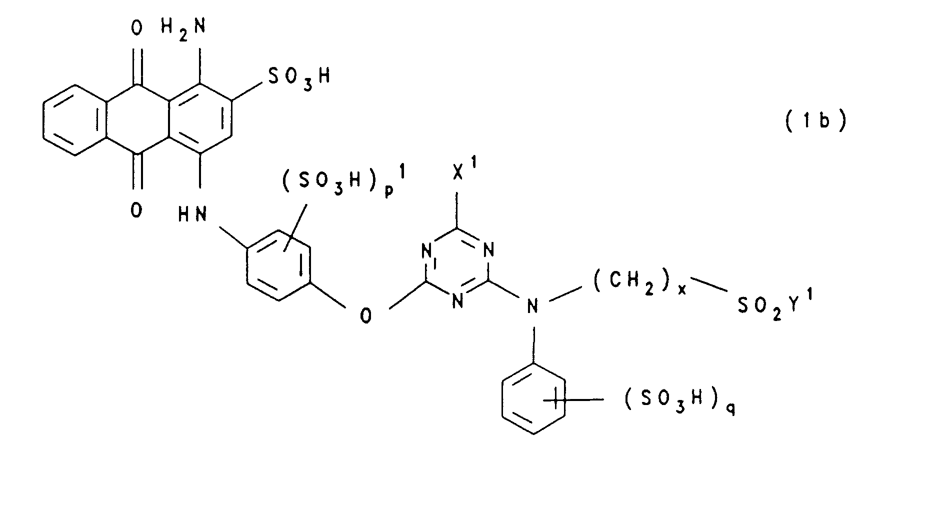 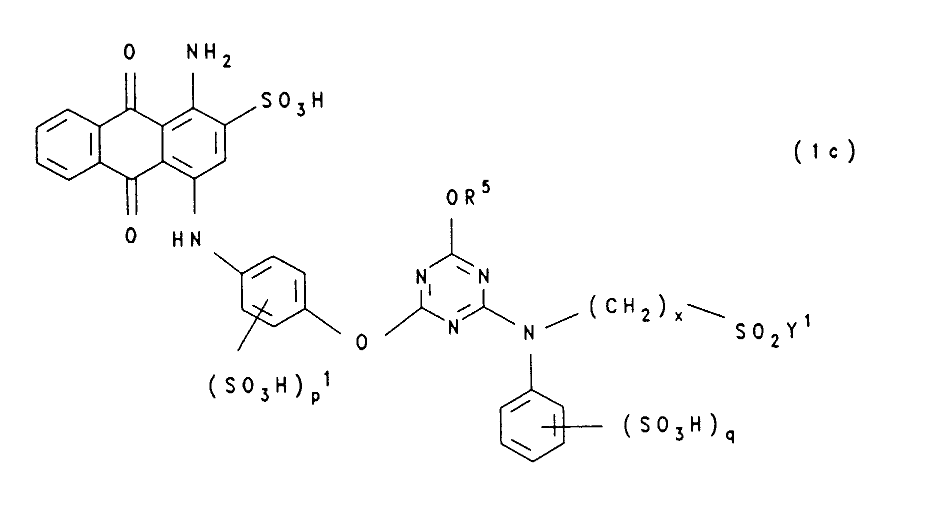 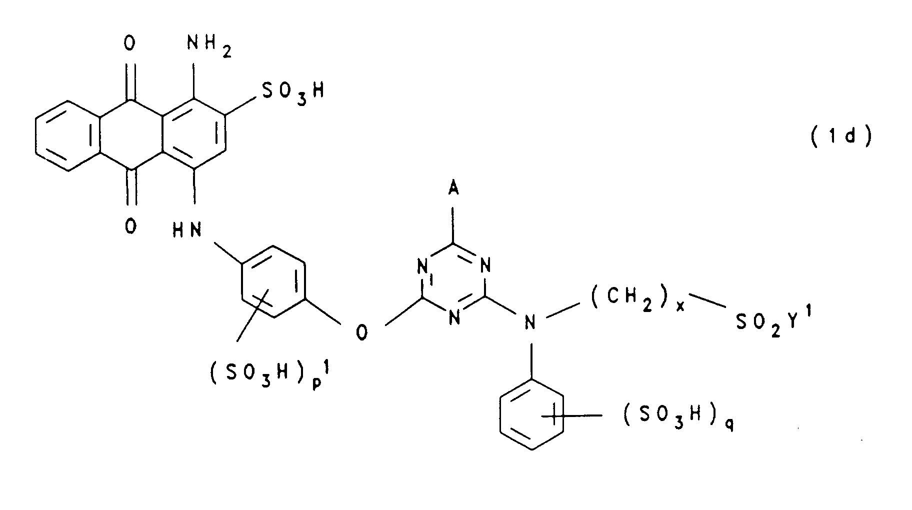 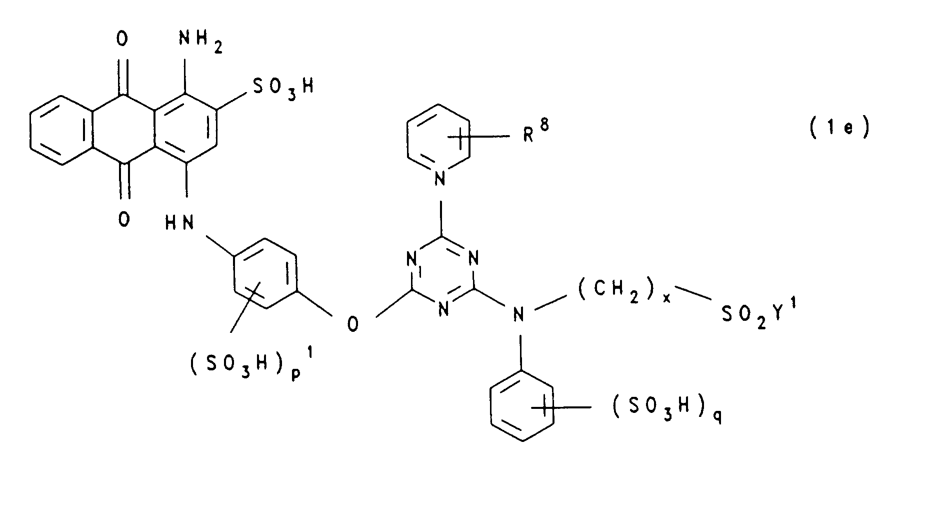 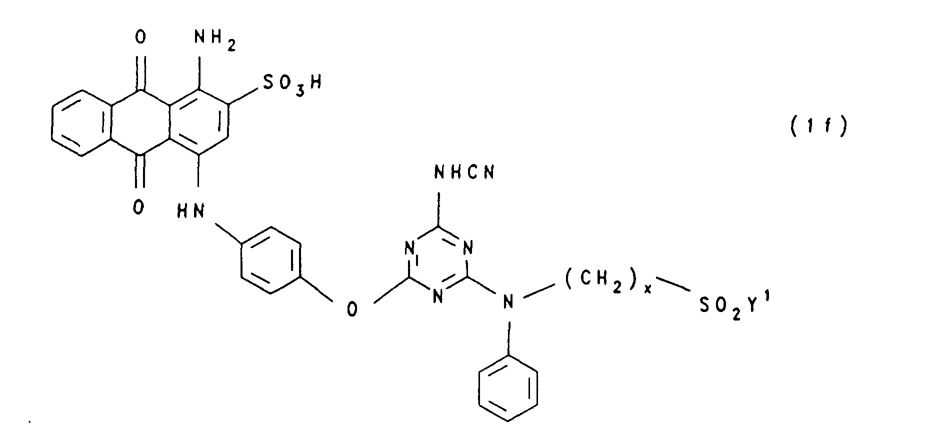 worin
x für 2 oder 3 steht;
X¹ Chlor oder Fluor ist;
R⁵ Methyl, Ethyl oder Phenyl ist;
A Amino, Carboxymethylamino, β-Carboxyethylamino, β-Sulfoethylamino, N-Methyl-β-sulfoethylamino, β-Sulfatoethylamino, β-Hydroxyethylamino, Bis-(β-hydroxyethyl)-amino, Morpholino, Piperidino, Pyrrolidino oder Trimethylammonium ist,
R⁸ Wasserstoff, Aminocarbonyl oder Carboxy ist; und
Y¹ β-Sulfatoethyl, β-Chlorethyl oder Vinyl ist.

9. Verfahren zur Herstellung einer Verbindung nach mindestens einem der Ansprüche 1 bis 8, dadurch gekennzeichnet, daß man eine Verbindung der Formel (2) 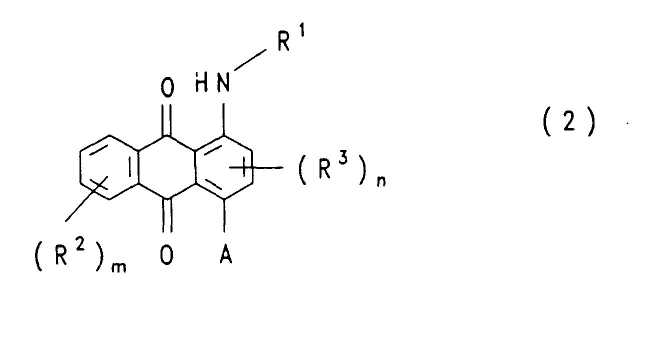 worin A für Fluor, Chlor, Brom, lod, Sulfo oder Nitro, insbesondere für Brom, steht,
mit einer Verbindung der Formel (3), 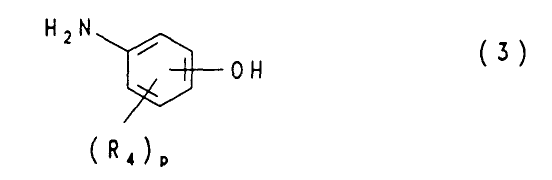 in Gegenwart einer Cu(I)- oder Cu(II)-Verbindung oder einer Mischung davon, zu einer Verbindung der Formel (4) 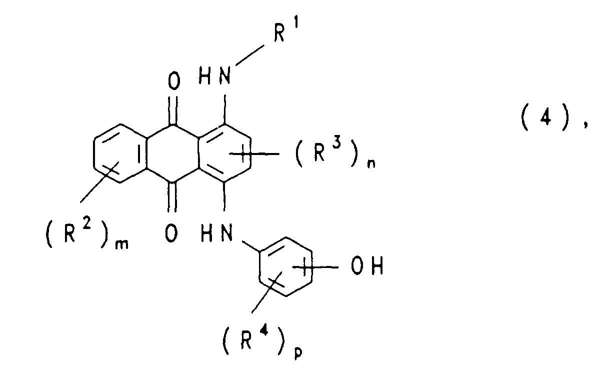 und die Verbindung der Formel (4) entweder mit einer Verbindung der Formel (5) 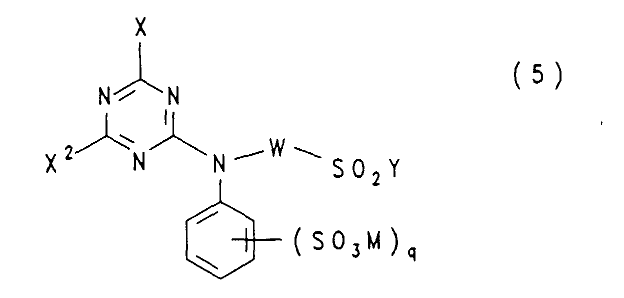 worin X² für Halogen steht, zur Verbindung der Formel (1) umsetzt; oder die Verbindung der Formel (4) zunächst mit einer Verbindung der Formel (6) 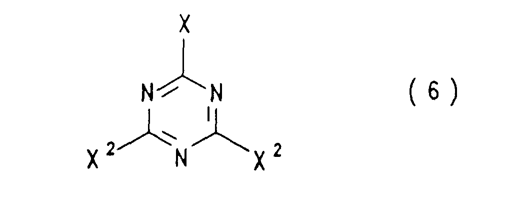 zu einer Verbindung der Formel (7) umsetzt 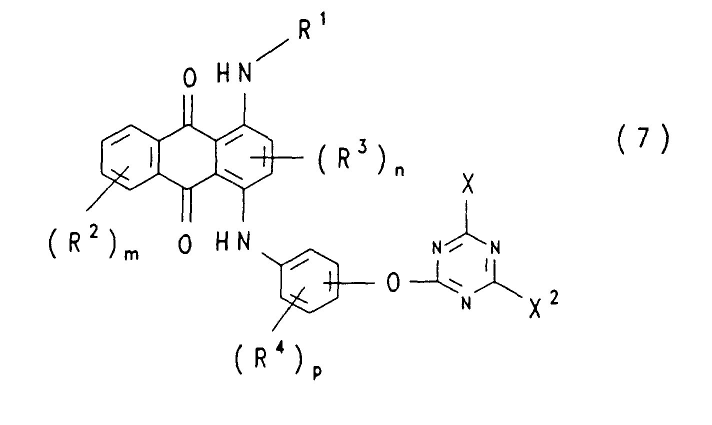 und die Verbindung der Formel (7) mit einem Amin der Formel (8) 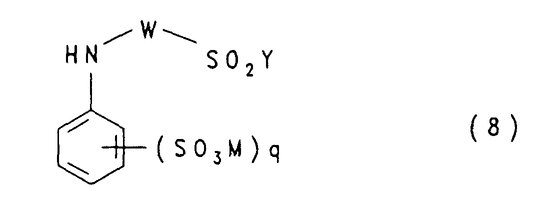 zur Verbindung der Formel (1) umsetzt.

10. Verfahren zur Herstellung einer Verbindung der Formel (4) 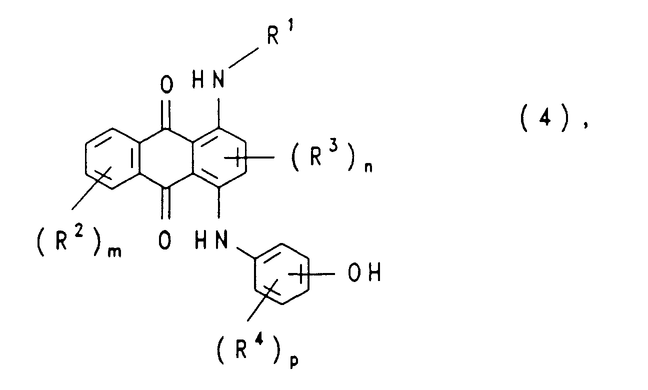 worin R¹, R², R³, R⁴, m, n und p eine der in Anspruch 1 genannten Bedeutungen haben, durch Kondensation einer Verbindung der Formel (2) 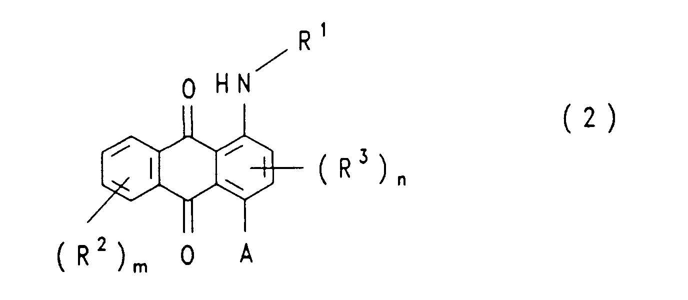 worin A für Fluor, Chlor, Brom, lod, Sulfo oder Nitro steht, mit einer Verbindung der Formel (3) 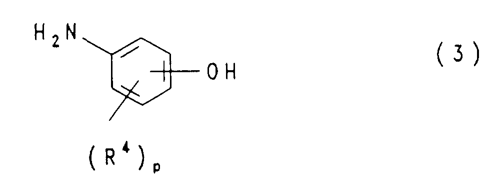 in Gegenwart einer Cu(II)-Verbindung.

11. Verfahren nach Anspruch 9 oder 10, dadurch gekennzeichnet, daß die Cu(II)-Verbindung Cu(II)-sulfat, Cu(II)-nitrat, Cu(II)-chlorid, Cu(II)-bromid, Cu(II)-carbonat oder Cu(II)-hydroxid ist.

12. Verfahren zur Herstellung einer Anthrachinonverbindung der Formel (1A) 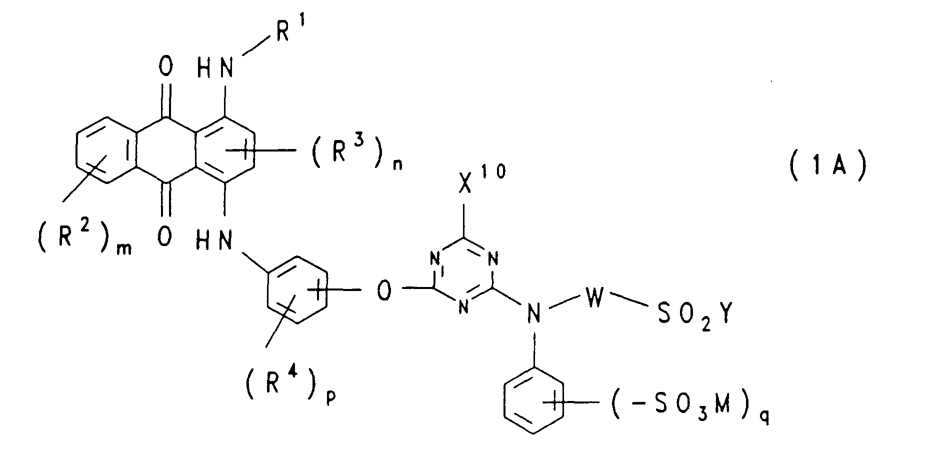 worin R¹, R², R³, R⁴, W, Y, M, m, n, p und q eine der in Anspruch 1 definierten Bedeutungen haben und X¹⁰ Amino, Cyanamino, Carboxy-(C₁-C₄)-alkyl-amino, C₁-C₄-Alkylamino, Di-(C₁-C₄-alkyl)amino, C₆-Arylamino, wobei die Alkyl- und Arylreste der besagten Alkylamino- und Arylamino-Gruppen unsubstituiert sind oder 1 bis 5 Substituenten aus der Gruppe Hydroxy, C₁-C₄-Alkoxy, Carboxy, Sulfo, Sulfato, C₁-C₄-Alkylsulfonyl, C₆-Arylsulfonyl, Halogen, Cyano und Nitro enthalten; oder der Rest eines heterocyclischen oder aliphatischen Amins, welches 1 bis 2 weitere Heteroatome aus der Gruppe N, O und S enthalten kann und mit einem Carboxyrest oder Aminocarbonylrest substituiert sein kann; oder die Gruppe -NR⁹R¹⁰R¹¹, worin R⁹, R¹⁰ und R¹¹ unabhängig voneinander Wasserstoff oder C₁-C₄-Alkyl bedeuten, ist,
dadurch gekennzeichnet, daß man eine Anthrachinonverbindung der Formel (1B) 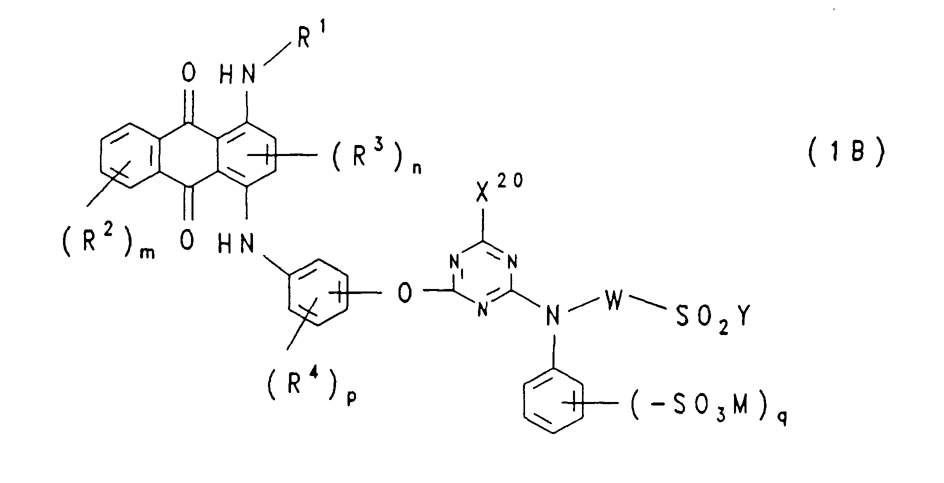 worin X²⁰ Chlor oder Fluor bedeutet,
mit Ammoniak, H₂NCN, einem Carboxy-(C₁-C₄)-alkylamin, C₁-C₄-Alkylamin, Di-(C₁-C₄-alkyl)-amin, C₆-Arylamin, wobei die Alkyl- und Arylreste der besagten Alkylamino- und Arylamino-Gruppen unsubstituiert sind oder 1 bis 5 Substituenten aus der Gruppe Hydroxy, C₁-C₄-Alkoxy, Carboxy, Sulfo, Sulfato, C₁-C₄-Alkylsulfonyl, C₆-Arylsulfonyl, Halogen, Cyano und Nitro enthalten;
oder mit einem heterocyclischen oder aliphatischen Amin, welches 1 bis 2 weitere Heteroatome aus der Gruppe N, O und S enthalten kann und mit einem Carboxyrest oder Aminocarbonylrest substituiert sein kann;
oder mit einem Mono-, Di- oder Tri-(C₁-C₄-alkyl)-ammonium-Salz umsetzt.

13. Verwendung einer Anthrachinonverbindung der Formel (1) gemäß mindestens einem der Ansprüche 1 bis 8 zum Färben oder Bedrucken eines hydroxy-, mercapto-, amino- oder carbonamidgruppenhaltigen oder eine Kombination dieser Gruppen enthaltenden Materials.

14. Verwendung gemäß Anspruch 13 zum Färben nach einem Textildruckverfahren oder nach einem kurzflottigen Klotzfärbeverfahren.

15. Verwendung einer der in Anspruch 8 definierten Verbindung der Formel (1a), (1c), (1d), (1e) oder (1f) nach Anspruch 13, dadurch gekennzeichnet, daß die genannten Farbstoffe auf die genannten Materialien im Ätzdruckverfahren appliziert werden.

## Claims

1. An anthraquinone compound of the formula (1), 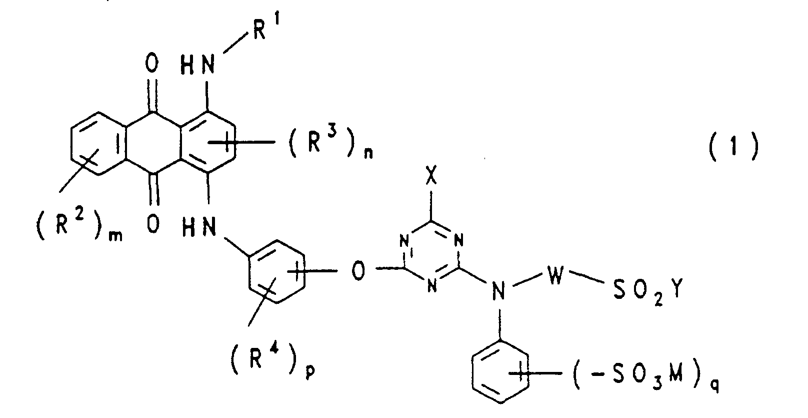 in which
R¹ is hydrogen, C₁-C₆-alkylcarbonyl, C₆-arylcarbonyl, C₁-C₆-alkyl, C₃-C₆-cycloalkyl or phenyl where C₁-C₆-alkyl, C₃-C₆-cycloalkyl and phenyl can be substituted by one or more radicals selected from the group consisting of hydroxyl, sulfo, carboxyl, C₁-C₄-alkyl, C₁-C₄-alkoxy, halogen, cyano, amino and nitro,
R² is sulfo or carboxyl,
m is 0 to 2,
R³ is sulfo, carboxyl or halogen,
n is 0 or 1,
R⁴ is sulfo, carboxyl, C₁-C₄-alkyl or C₁-C₄-alkoxy,
p is 0 to 2,
W is a C₁-C₆-alkylene group,
Y is vinyl or -CH₂CH₂-L where L is a group which can be eliminated under alkaline conditions,
M is hydrogen or an alkali metal or a stoichiometric equivalent of an alkaline earth metal,
q is 0 or 1, and
x is halogen, hydroxyl, C₁-C₄-alkyloxy, C₆-aryloxy, cyanamino, amino, carboxy-(C₁-C₄)-alkylamino, C₁-C₄-alkylamino, di-(C₁-C₄-alkyl)amino or C₆-arylamino where the alkyl and aryl radicals of the alkylamino and arylamino groups mentioned are unsubstituted or contain 1 to 5 substituents selected from the group consisting of hydroxyl, C₁-C₄-alkoxy, carboxyl, sulfo, sulfato, C₁-C₄-alkylsulfonyl, C₆-arylsulfonyl, halogen, cyano and nitro, or is the radical of a heterocyclic or aliphatic amine which may contain 1 or 2 further hetero atoms selected from the group consisting of N, O and S and can be substituted by a carboxyl radical or an aminocarbonyl radical, or is the group -NR⁹R¹⁰R¹¹ in which R⁹, R¹⁰ and R¹¹, independently of one another, are hydrogen or C₁-C₄-alkyl.

2. An anthraquinone compound as claimed in claim 1, wherein
R¹ is hydrogen, C₁-C₄-alkyl, C₅-C₆-cycloalkyl, acetyl, benzoyl, phenyl or methylphenylcarbonyl or C₁-C₄-alkyl, C₅-C₆-cycloalkyl or phenyl each of which is substituted by 1 to 3 radicals selected from the group consisting of hydroxyl, sulfo, carboxyl, methyl, ethyl, methoxy, ethoxy, fluorine, chlorine, bromine, cyano or nitro.

3. An anthraquinone compound as claimed in claim 1 or 2, wherein R³ is chlorine, bromine or a sulfo group which is in the ortho position relative to the NH-R¹ group.

4. An anthraquinone compound as claimed in at least one of claims 1 to 3, wherein W is C₂-C₃-alkylene, preferably 1,2-ethylene or 1,3-propylene.

5. An anthraquinone compound as claimed in at least one of claims 1 to 4, wherein L is chlorine, bromine, -OSO₃M, -SSO₃M or -OPO₃M₂ where M is hydrogen or an alkali metal.

6. An anthraquinone compound as claimed in at least one of claims 1 to 5, wherein
m is 0,
n is 1,
p is 0 or 1, and
q is 0.

7. An anthraquinone compound as claimed in at least one of claims 1 to 6, wherein
X is fluorine, chlorine, cyanamino, amino, carboxymethylamino, β-carboxyethylamino, β-sulfoethylamino, N-methyl-β-sulfoethylamino, β-sulfatoethylamino, β-hydroxyethylamino, bis (β-hydroxyethyl) amino, pyrrolidino, piperidino, piperazino, morpholino, pyridin-1-yl, 3-carboxypyridin-1-yl, 3-aminocarbonylpyridin-1-yl or trimethylammonium.

8. An anthraquinone compound as claimed in claim 1, wherein the anthraquinone compound has the formula (1a), (1b), (1c), (1d), (le) or (1f) 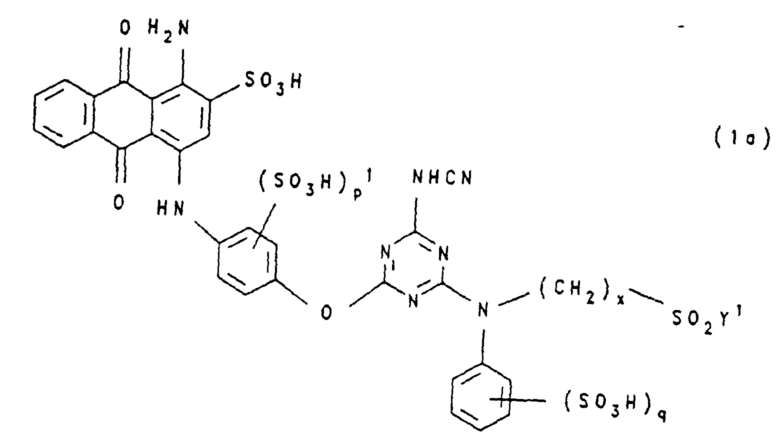 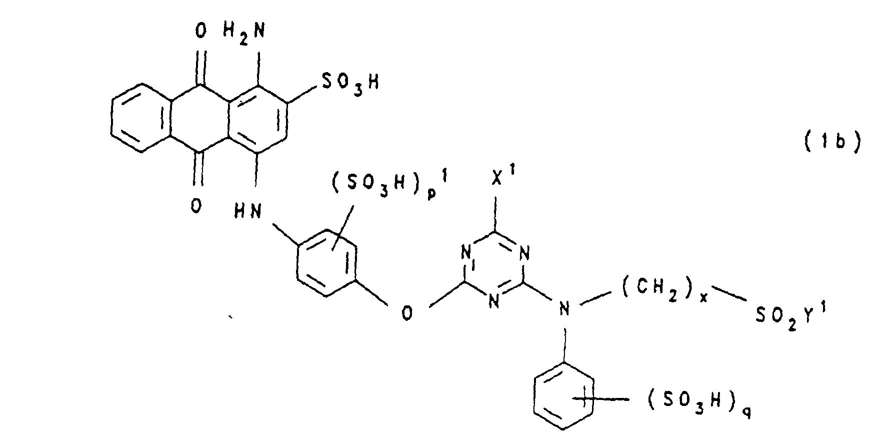 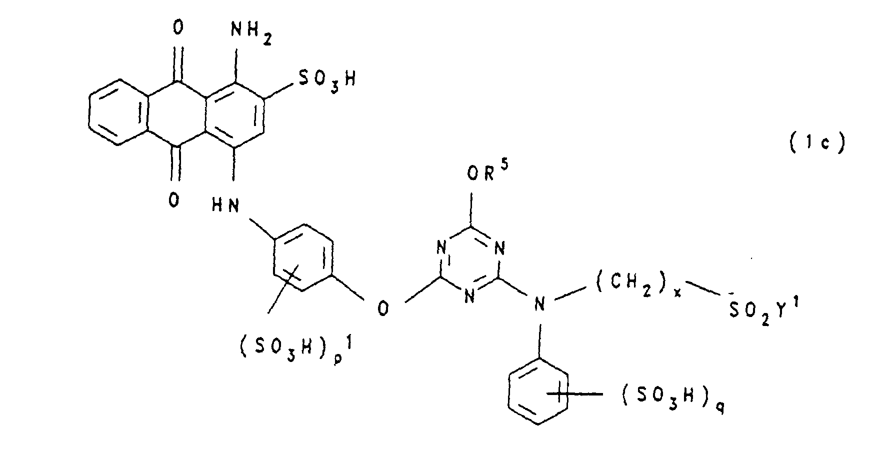 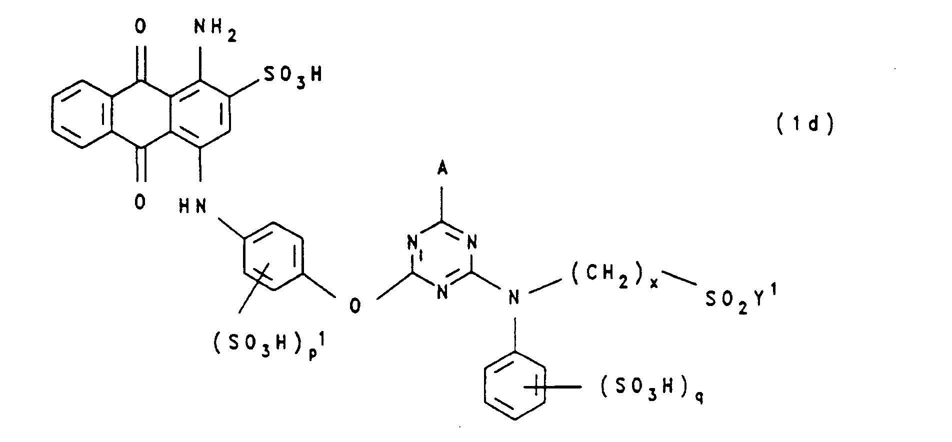 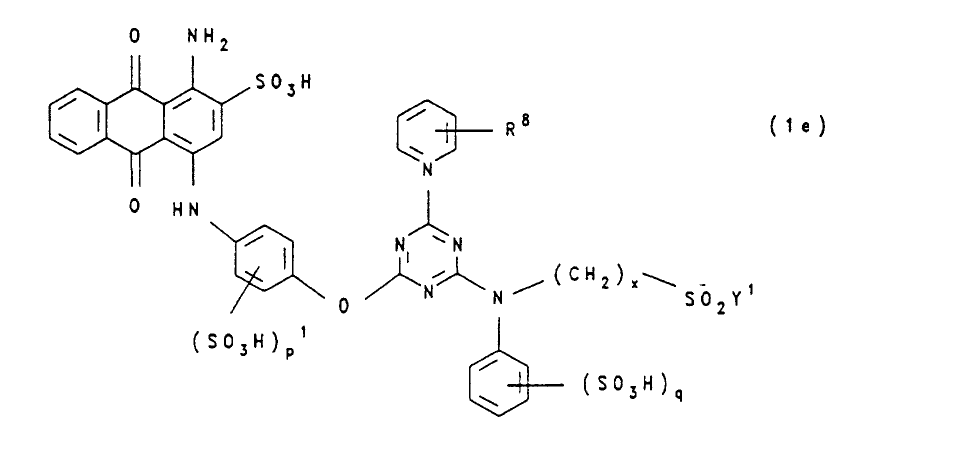 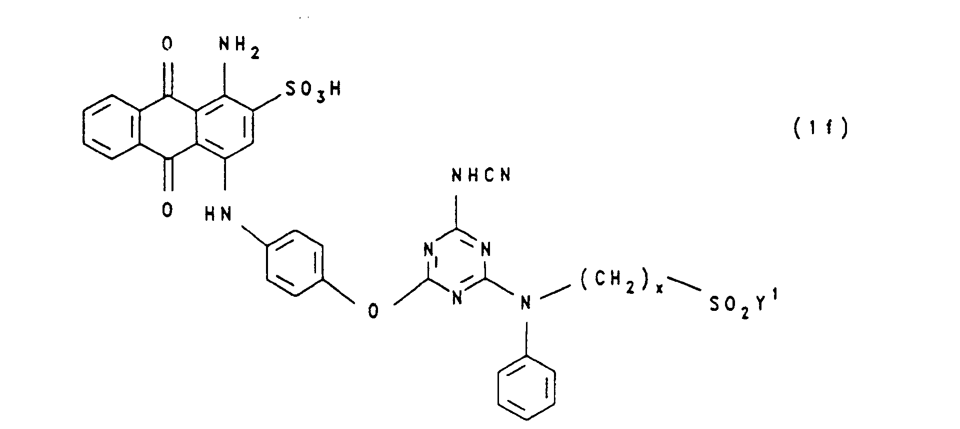 in which
x is 2 or 3,
X¹ is chlorine or fluorine,
R⁵ is methyl, ethyl or phenyl,
A is amino, carboxymethylamino, β-carboxyethylamino, β-sulfoethylamino, N-methyl-β-sulfoethylamino, β-sulfatoethylamino, β-hydroxyethylamino, bis (β-hydroxyethyl)amino, morpholino, piperidino, pyrrolidino or trimethylammonium,
R⁸ is hydrogen, aminocarbonyl or carboxyl, and
Y¹ is β-sulfatoethyl, β-chloroethyl or vinyl.

9. A process for preparing a compound as claimed in at least one of claims 1 to 8, which process comprises reacting a compound of the formula (2) 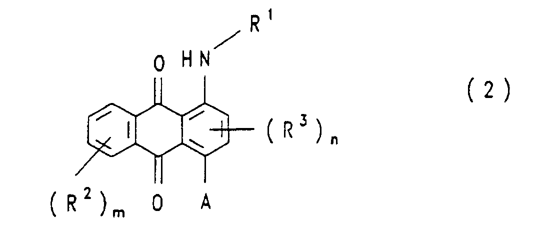 in which A is fluorine, chlorine, bromine, iodine, sulfo or nitro, in particular bromine,
with a compound of the formula (3) 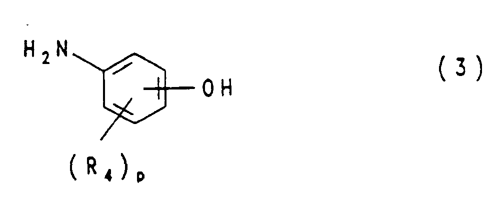 in the presence of a copper(I) or a copper(II) compound or a mixture thereof, to give a compound of the formula (4) 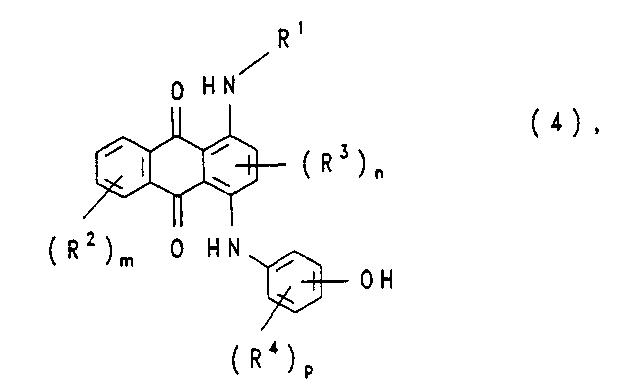 and reacting the compound of the formula (4) either with a compound of the formula (5) 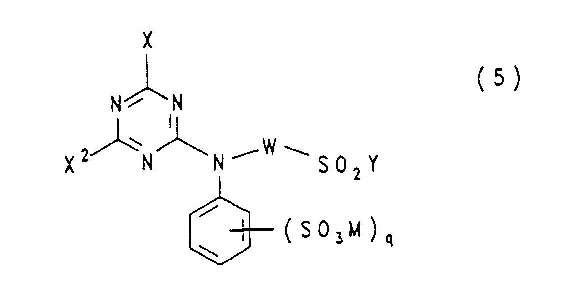 in which X² is halogen, to give the compound of the formula (1), or reacting the compound of the formula (4) first with a compound of the formula (6) 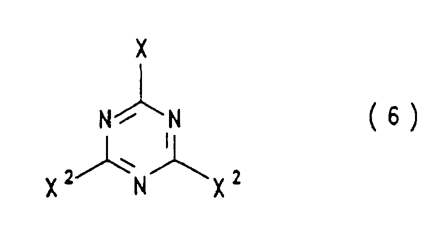 to give a compound of the formula (7) 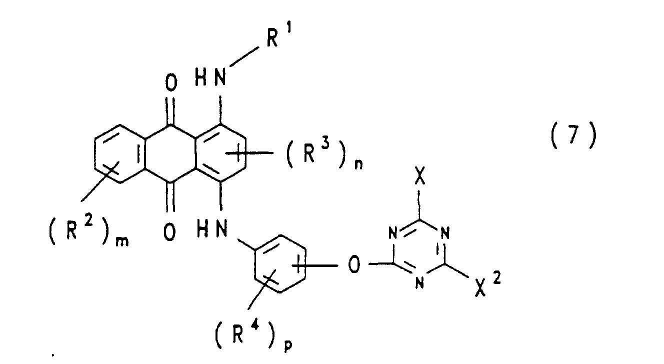 and reacting the compound of the formula (7) with an amine of the formula (8) 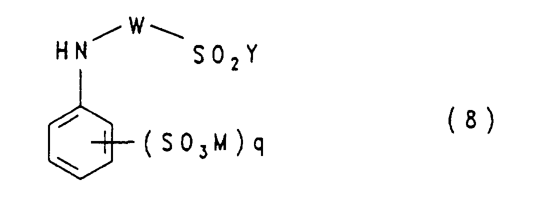 to give the compound of the formula (1).

10. A process for preparing a compound of the formula (4) 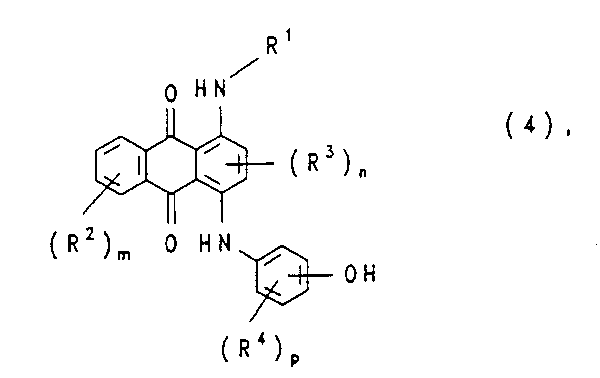 in which R¹, R², R³, R⁴, m, n and p have one of the meanings mentioned in claim 1, by condensing a compound of the formula (2) 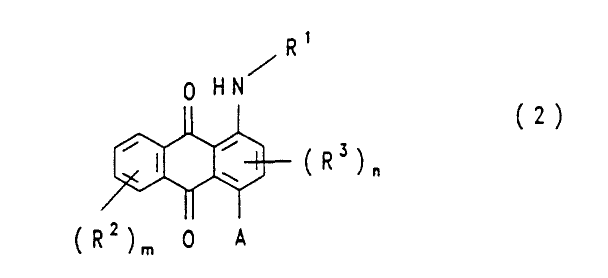 in which A is fluorine, chlorine, bromine, iodine, sulfo or nitro, with a compound of the formula (3) 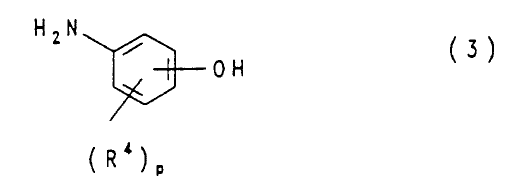 in the presence of a copper(II) compound.

11. The process as claimed in claim 9 or 10, wherein the copper(II) compound is copper(II) sulfate, copper(II) nitrate, copper(II) chloride, copper(II) bromide, copper(II) carbonate or copper(II) hydroxide.

12. A process for preparing an anthraquinone compound of the formula (1A) 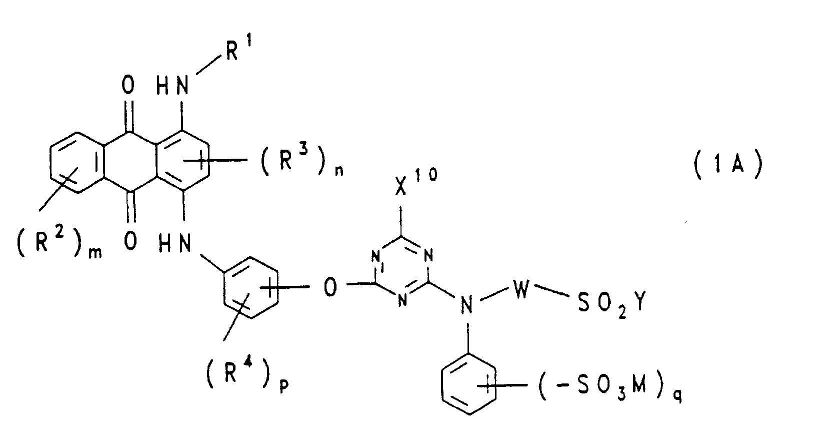 in which R¹, R², R³, R⁴, W, Y, M, m, n, p and q have one of the meanings defined in claim 1 and X¹⁰ is amino, cyanamino, carboxy-(C₁-C₄) -alkylamino, C₁-C₄-alkylamino, di-(C₁-C₄-alkyl)amino, C₆-arylamino where the alkyl and aryl radicals of the alkylamino and arylamino groups mentioned are unsubstituted or contain 1 to 5 substituents selected from the group consisting of hydroxyl, C₁-C₄-alkoxy, carboxyl, sulfo, sulfato, C₁-C₄-alkylsulfonyl, C₆-arylsulfonyl, halogen, cyano and nitro, or is the radical of a heterocyclic or aliphatic amine, which may contain 1 to 2 further hetero atoms selected from the group consisting of N, O and S and can be substituted by a carboxyl radical or an aminocarbonyl radical, or is the group -NR⁹R¹⁰R¹¹ in which R⁹, R¹⁰ and R¹¹, independently of one another, are hydrogen or C₁-C₄-alkyl,
which process comprises reacting an anthraquinone compound of the formula (1B) 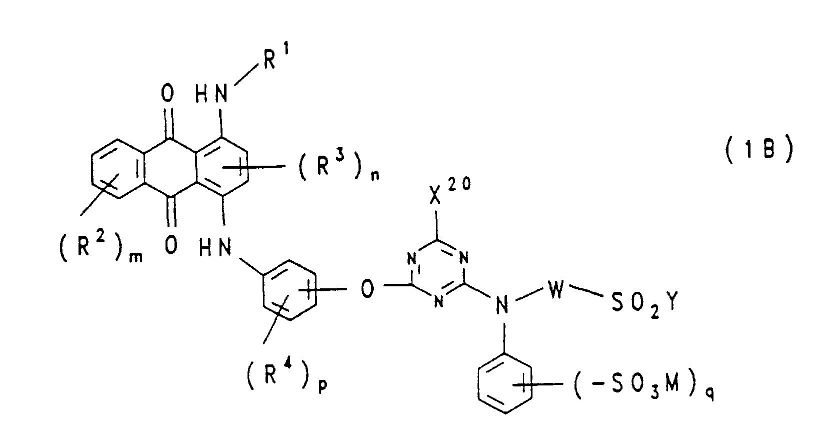 in which X²⁰ is chlorine or fluorine,
with ammonia, H₂NCN, a carboxy-(C₁-C₄)-alkylamine, C₁-C₄-alkylamine, di-(C₁-C₄-alkyl)amine, C₆-arylamine where the alkyl and aryl radicals of the alkylamino and arylamino groups mentioned are unsubstituted or contain 1 to 5 substituents selected from the group consisting of hydroxyl, C₁-C₄-alkoxy, carboxyl, sulfo, sulfato, C₁-C₄-alkylsulfonyl, C₆-arylsulfonyl, halogen, cyano and nitro,
or with a heterocyclic or aliphatic amine, which may contain 1 to 2 further hetero atoms selected from the group consisting of N, O and S and can be substituted by a carboxyl radical or an aminocarbonyl radical,
or with a mono-, di- or tri-(C₁-C₄-alkyl)ammonium salt.

13. Use of an anthraquinone compound of the formula (1) as claimed in at least one of claims 1 to 8 for dyeing or printing a hydroxyl-, mercapto-, amino- or carboxamido-containing material or a material containing a combination of these groups.

14. Use as claimed in claim 13 for dyeing using a textile printing method or a short-liquor pad-dyeing method.

15. Use as claimed in claim 13 of a compound of the formula (1a), (1c), (1d), (1e) or (1f) defined in claim 8, wherein the dyes mentioned are applied to the materials mentioned by the discharge printing method.

## Revendications

1. Composé anthraquinone de formule (1) 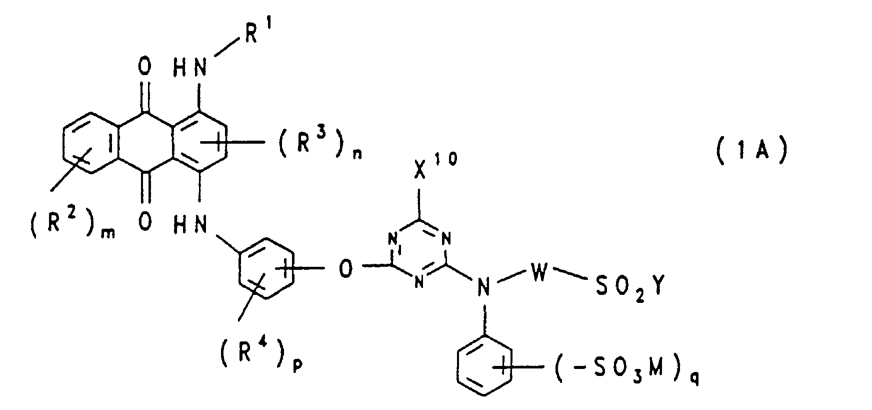 dans laquelle
R¹ représente un hydrogène, un C₁-C₆-alkylcarbonyle, un C₆-arylcarbonyle, un alkyle en C₁₋C₆, un cycloalkyle en C₃-C₆ ou un phényle, où l'alkyle en C₁-C₆, le cycloalkyle en C₃-C₆ et le phényle peuvent être substitués par un ou plusieurs radicaux parmi le groupe constitué d'un hydroxy, d'un sulfo, d'un carboxy, d'un alkyle en C₁-C₄, d'un alcoxy en C₁-C₄, d'un halogène, d'un cyano, d'un amino et d'un nitro ;
R² représente un sulfo ou un carboxy ;
m représente un nombre de 0 à 2 ;
R³ représente un sulfo, un carboxy ou un halogène ;
n représente le nombre 0 ou 1 ;
R⁴ représente un sulfo, un carboxy, un alkyle en C₁-C₄ ou un alcoxy en C₁-C₄ ;
p représente un nombre de 0 à 2 ;
w représente un groupe alkylène en C₁-C₆ ;
Y représente un vinyle ou -CH₂CH₂-L, où L est un groupe pouvant être éliminé dans des conditions alcalines ;
M représente un hydrogène ou un métal alcalin ou un équivalent stoechiométrique d'un métal alcalino-terreux ;
q représente le nombre 0 ou 1 ; et
x représente un halogène, un hydroxy, un alkyloxy en C₁-C₄, un aryloxy en C₆, un cyanamino, un amino, un carboxy-(C₁-C₄)-alkylamino, un C₁-C₄-alkylamino, un di-(C₁-C₄-alkyl) amino ou un C₆-arylamino, où les radicaux alkyle et aryle desdits groupes alkylamino et arylamino sont non substitués ou renferment de 1 à 5 substituants parmi le groupe constitué d'un hydroxy, d'un alcoxy en C₁-C₄, d'un carboxy, d'un sulfo, d'un sulfato, d'un C₁-C₄-alkylsulfonyle, d'un C₆-arylsulfonyle, d'un halogène, d'un cyano et d'un nitro ; ou le radical d'une amine hétérocyclique ou aliphatique, qui peut renfermer de 1 à 2 hétéroatomes supplémentaires parmi le groupe constitué de N, de O et de S et peut être substitué par un radical carboxy ou un radical aminocarbonyle ; ou le groupe -NR⁹R¹⁰R¹¹, où R⁹, R¹⁰ et R¹¹ représentent, indépendamment les uns des autres, un hydrogène ou un alkyle en C₁-C₄.

2. Composé anthraquinone selon la revendication 1,
caractérisé en ce que
R¹ représente un hydrogène, un alkyle en C₁-C₄, un cycloalkyle en C₅-C₆, un acétyle, un benzoyle, un phényle ou méthylphénylcarbonyle, ou un alkyle en C₁-C₄, un cycloalkyle en C₅-C₆ et un phényle qui sont substitués par 1 à 3 radicaux parmi le groupe constitué d'un hydroxy, d'un sulfo, d'un carboxy, d'un méthyle, d'un éthyle, d'un méthoxy, d'un éthoxy, d'un fluor, d'un chlore, d'un brome, d'un cyano ou d'un nitro.

3. Composé anthraquinone selon la revendication 1 ou 2, caractérisé en ce que R³ est un chlore, un brome ou un groupe sulfo en position ortho par rapport au groupe NH-R¹.

4. Composé anthraquinone selon au moins l'une des revendications 1 à 3, caractérisé en ce que W est un alkylène en C₂-C₃, de préférence un 1,2-éthylène ou un 1,3-propylène.

5. Composé anthraquinone selon au moins l'une des revendications 1 à 4, caractérisé en ce que L est un chlore, un brome, -OSO₃M, -SSO₃M ou -OPO₃M₂, où M représente un hydrogène ou un métal alcalin.

6. Composé anthraquinone selon au moins l'une des revendications 1 à 5, caractérisé en ce que
m est le nombre 0 ;
n est le nombre 1 ;
p est le nombre 0 ou 1 et
q est le nombre 0.

7. Composé anthraquinone selon au moins l'une des revendications 1 à 6, caractérisé en ce que
X représente un fluor, un chlore, un cyanoamino, un amino, un carboxyméthylamino, un β-carboxyéthylamino, un β-sulfoéthylamino, un N-méthyl-β-sulfoéthylamino, un β-sulfatoéthylamino, un β-hydroxyéthylamino, un bis(β-hydroxyéthyl) amino, un pyrrolidino, un pipéridino, un pipérazino, un morpholino, un pyridin-1-yle, un 3-carboxypyridin-1-yle, un 3-aminocarbonylpyridin-1-yle ou un triméthylammonium.

8. Composé anthraquinone selon la revendication 1, caractérisé par les formules (1a), (1b), (1c), (1d), (1e) ou (1f) 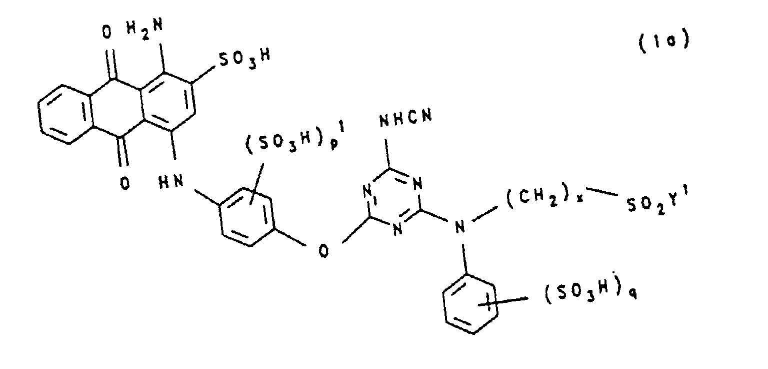 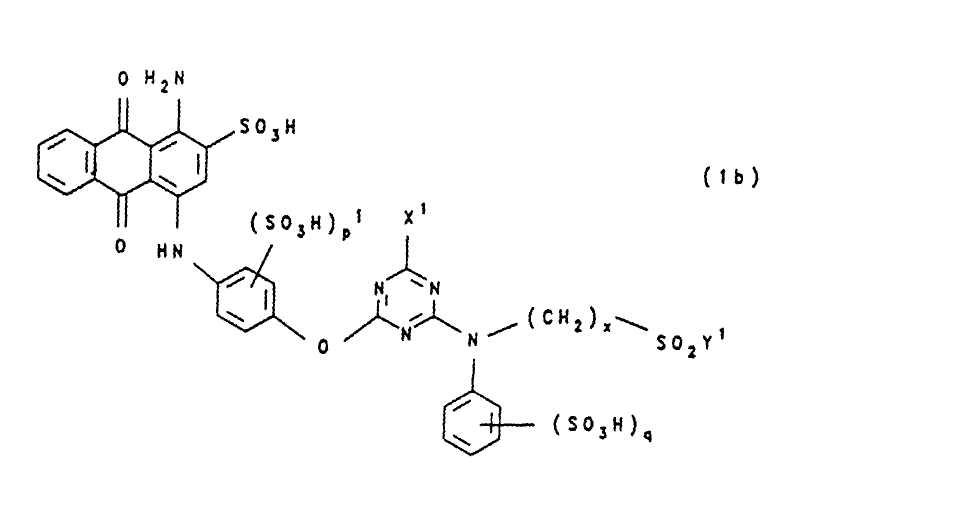 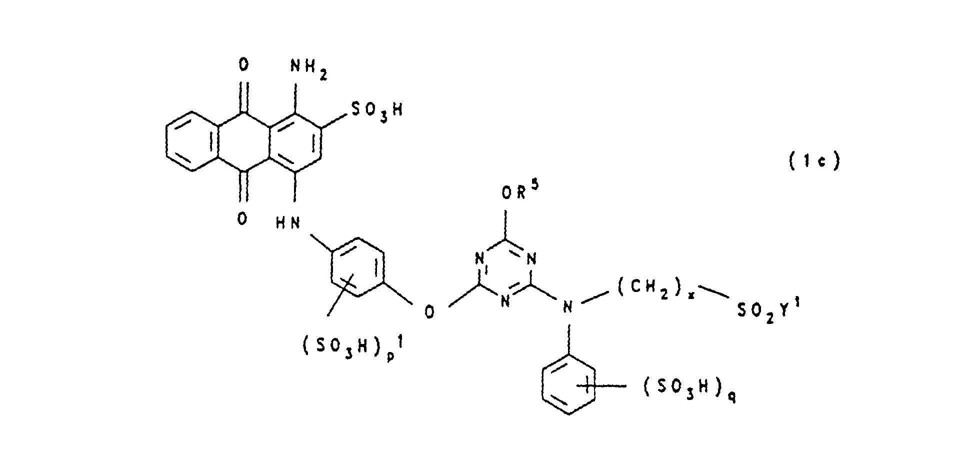 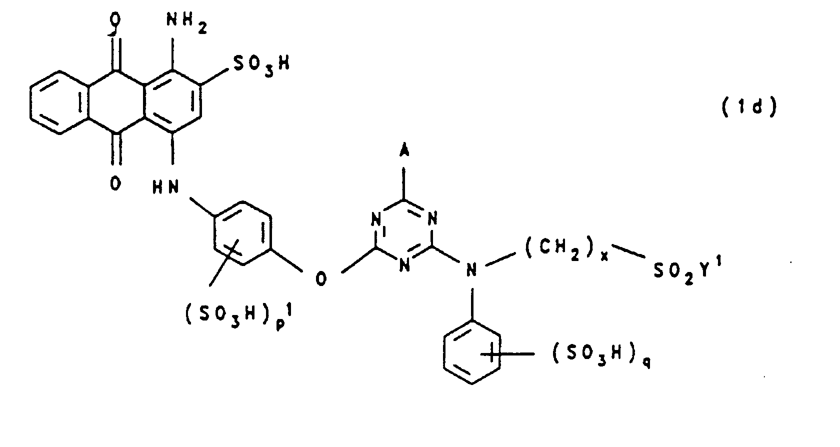 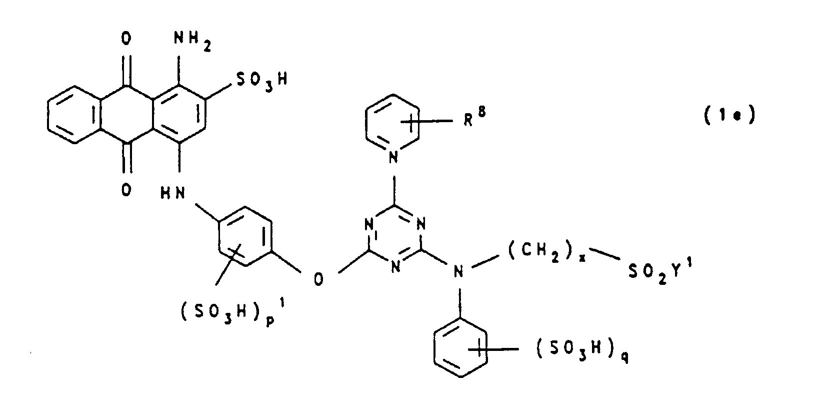 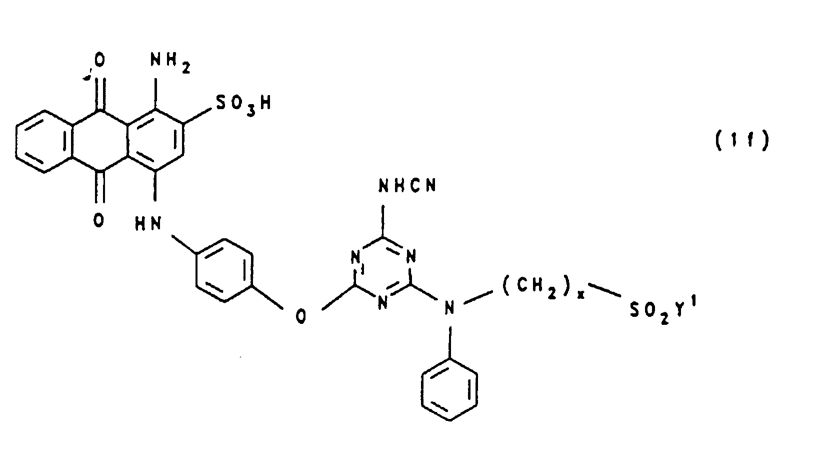 dans lesquelles
x vaut 2 ou 3 ;
X¹ est un chlore ou un fluor ;
R⁵ est un méthyle, un éthyle ou un phényle ;
A est un amino, un carboxyméthylamino, un β-carboxyéthylamino, un β-sulfoéthylamino, un N-méthyl-β-sulfoéthylamino, un β-sulfatoéthylamino, un β-hydroxyéthylamino, un bis (β-hydroxyéthyl) amino, un morpholino, un pipéridino, un pyrrolidino ou un triméthylammonium,
R⁸ est un hydrogène, un aminocarbonyle ou un carboxy ; et
Y¹ est un β-sulfatoéthyle, un β-chloroéthyle ou un vinyle.

9. Procédé de préparation d'un composé selon au moins l'une des revendications 1 à 8, caractérisé en ce que l'on fait réagir un composé de formule (2) 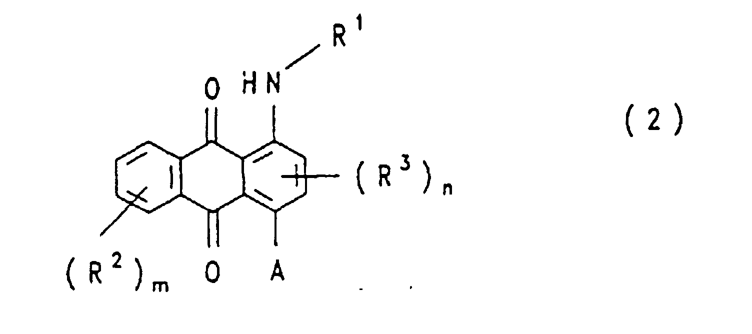 dans laquelle A désigne un fluor, un chlore, un brome, un iode, un sulfo ou un nitro, en particulier un brome, avec un composé de formule (3), 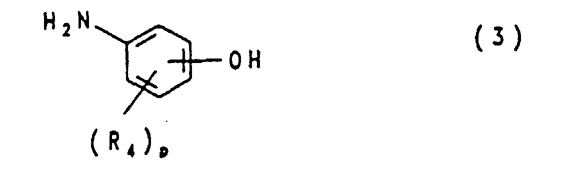 en présence d'un composé de Cu(I) ou Cu(II) ou un mélange de ceux-ci, pour donner lieu à un composé de formule (4) 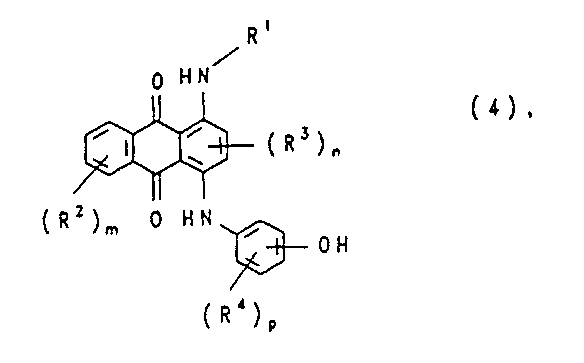 et on fait réagir le composé de formule (4) soit avec un composé de formule (5) 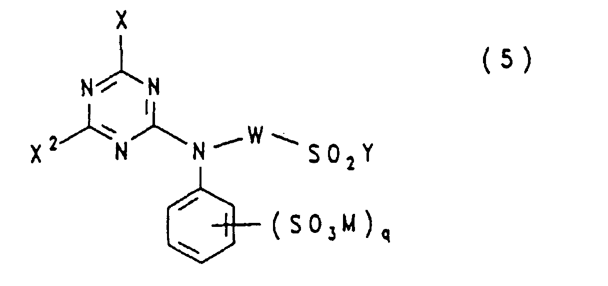 dans laquelle X² désigne un halogène, pour donner lieu au composé de formule (1) ;
soit on fait réagir le composé de formule (4) d'abord avec un composé de formule (6) 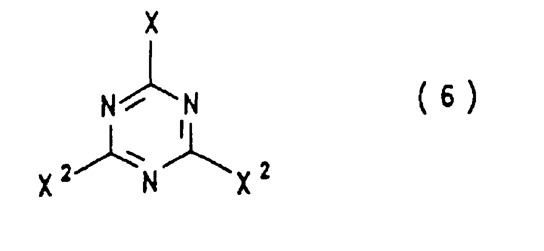 pour donner lieu à un composé de formule (7) 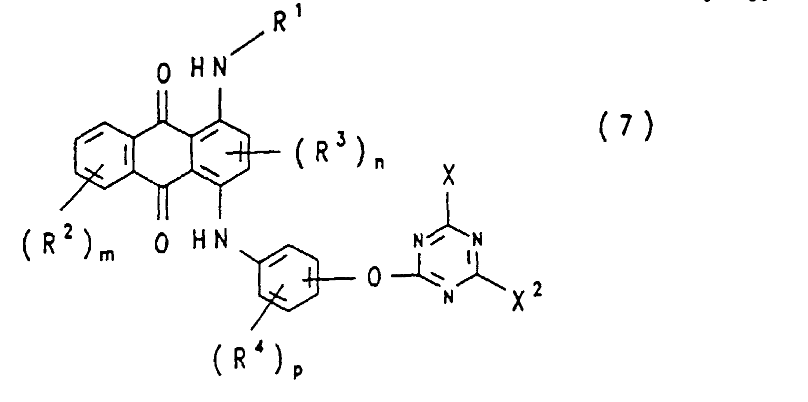 et on fait réagir le composé de formule (7) avec une amine de formule (8) 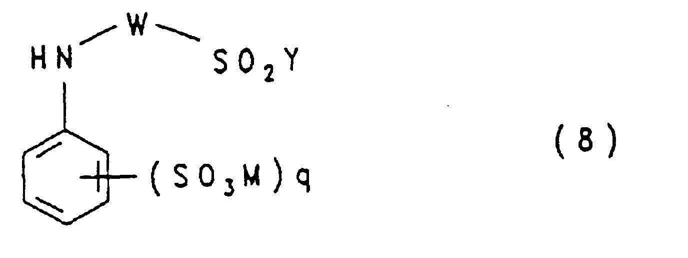 pour donner lieu au composé de formule (1).

10. Procédé de préparation d'un composé de formule (4) 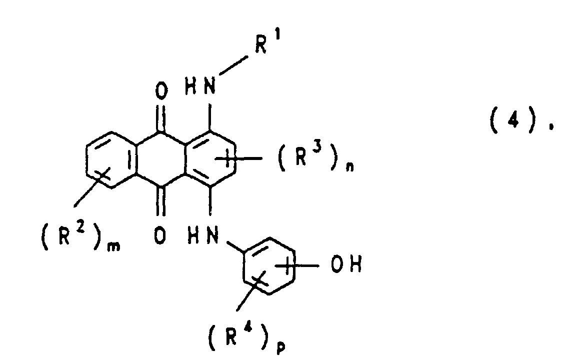 dans laquelle R¹, R², R³, R⁴, m, n et p ont l'une des significations mentionnées à la revendication 1, par condensation d'un composé de formule (2) 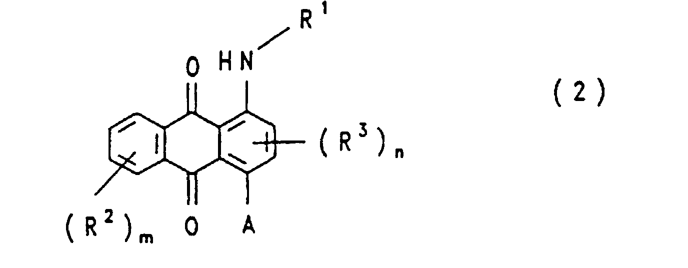 dans laquelle A désigne un fluor, un chlore, un brome, un iode, un sulfo ou un nitro, avec un composé de formule (3) 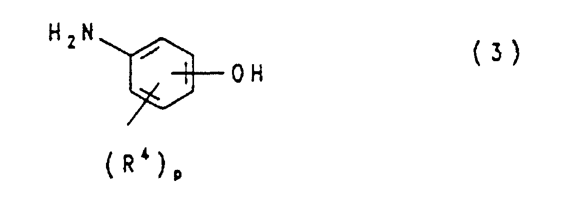 en présence d'un composé de Cu(II).

11. Procédé selon la revendication 9 ou 10, caractérisé en ce que le composé de Cu(II) est le sulfate de Cu(II), le nitrate de Cu(II), le chlorure de Cu(II), le bromure de Cu(II), le carbonate de Cu(II) ou l'hydroxyde de Cu(II).

12. Procédé de préparation d'un composé anthraquinone de formule (1A) 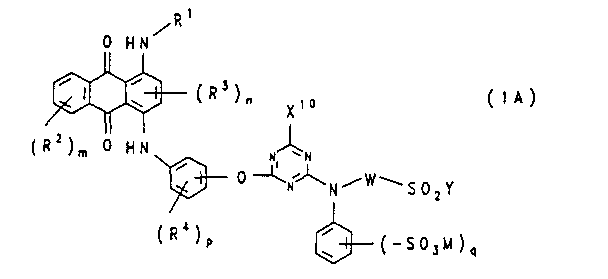 dans laquelle R¹, R², R³, R⁴, W, Y, M, m, n, p et q ont l'une des significations définies à la revendication 1, et X¹⁰ représente un amino, un cyanamino, un carboxy(C₁-C₄)-alkylamino, un C₁-C₄-alkylamino, un di-(C₁-C₄-alkyl)amino, un C₆-arylamino, où les radicaux alkyle et aryle desdits groupes alkylamino et arylamino sont non substitués ou renferment de 1 à 5 substituants parmi le groupe constitué d'un hydroxy, d'un alcoxy en C₁-C₄, d'un carboxy, d'un sulfo, d'un sulfato, d'un C₁-C₄-alkylsulfonyle, d'un C₆-arylsulfonyle, d'un halogène, d'un cyano et d'un nitro ; ou le radical d'une amine hétérocyclique ou aliphatique, qui peut renfermer de 1 à 2 hétéroatomes supplémentaires parmi le groupe constitué de N, de O et de S et peut être substitué par un radical carboxy ou un radical aminocarbonyle ; ou le groupe -NR⁹R¹⁰R¹¹, où R⁹, R¹⁰ et R¹¹ représentent, indépendamment les uns des autres, un hydrogène ou un alkyle en C₁-C₄,
caractérisé en ce que l'on fait réagir un composé anthraquinone de formule (1B) 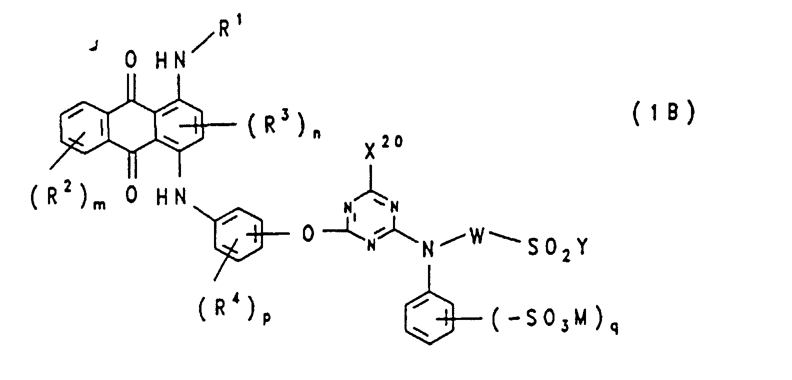 dans laquelle X²⁰ représente un chlore ou un fluor,
avec de l'ammoniac, H₂NCN, une carboxy-(C₁-C₄)-alkylamine, une C₁-C₄-alkylamine, une di (C₁-C₄-alkyl)amine, une C₆-arylamine, où les radicaux alkyle et aryle desdits groupes alkylamino et arylamino sont non substitués ou renferment de 1 à 5 substituants parmi le groupe constitué d'un hydroxy, d'un alcoxy en C₁-C₄, d'un carboxy, d'un sulfo, d'un sulfato, d'un C₁-C₄-alkylsulfonyle, d'un C₆-arylsulfonyle, d'un halogène, d'un cyano et d'un nitro ;
ou avec une amine hétérocyclique ou aliphatique qui peut renfermer de 1 à 2 hétéroatomes supplementaires parmi le groupe constitué de N, de O et de S et peut être substituée par un radical carboxy ou un radical aminocarbonyle ;
ou avec un sel de mono-, di- ou tri-(C₁-C₄-alkyl)ammonium.

13. Utilisation d'un composé anthraquinone de formule (1) selon au moins l'une des revendications 1 à 8, pour la teinture ou l'impression d'une matière renfermant des groupes hydroxy, mercapto, amino ou carboxamide ou renfermant une combinaison de ces groupes.

14. Utilisation selon la revendication 13, pour la teinture conformément à un procédé d'impression textile ou conformément à un procédé de teinture par foulardage en bain court.

15. Utilisation d'un composé défini à la revendication 8, de formule (1a), (1c), (1d), (1e) ou (1f) selon la revendication 13, caractérisée en ce que les colorants mentionnés sont appliqués sur les matières mentionnées dans le procédé d'impression par mordançage.
